(19)

Europäisches Patentamt

European Patent Office

Office européen des brevets

(11) **EP 3 026 435 A1**

(12) **EUROPEAN PATENT APPLICATION**

(43) Date of publication:
01.06.2016 Bulletin 2016/22

(51) Int Cl.:
*G01N 33/68* *(2006.01)*        *G01N 33/84* *(2006.01)*

(21) Application number: 14195383.6

(22) Date of filing: 28.11.2014

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: **Fundación Para El Fomento De La Investigación Sanitaria Y Biomédica De La Comunitat Valenciana**
**46010 Valencia (ES)**

(72) Inventors:
• **Mira Obrador, Alejandro**
**46540 Valencia (ES)**
• **Soro Áurea, Simón**
**46820 Valencia (ES)**

(74) Representative: **Illescas, Manuel**
**Manuel Illescas y Asocioados, S.L. (MIA)**
**Principe de Vergara n° 197**
**Oficina 1°A**
**28002 Madrid (ES)**

(54) **Diagnostic prediction of caries risk by saliva marker analysis**

(57)    The present invention discloses a method for predicting caries risk in a subject and a kit based on said method, characterized in that said method comprises:
(a) determining the concentration in saliva from said subject of at least one compound selected from each of salivary components (i) of the immune system, (ii) involved in microbial adhesion to a surface of the buccal cavity; and (iii) involved in metabolism in the buccal cavity or in regulating the pH thereof;
(b) comparing a response variable calculated from more than one concentration determined in step (a) with a corresponding reference response variable in a population of subjects without caries or in a population of subjects with caries; and
(c) determining caries risk based on the degree of difference in response variable compared in step (b), wherein a greater degree of difference between the response variable of the subject and the corresponding reference response variable in a population of subjects without caries or in a population of subjects with caries, is indicative of higher caries risk in said subject.

Figure 11

EP 3 026 435 A1

## Description

### Field of the Invention

The present invention may be included in the field of the medicine in general, more particularly in the field of dental care and oral health.

Thus the present invention is focused on a method and kit for predicting the risk that a subject develops caries. More specifically, the present invention refers to the identification of specific markers in a saliva sample which can be used as an indicator of caries risk. Furthermore, the present invention also discloses the use of the method or the kit in predicting caries risk in a subject and/or in prescribing a plan of care which reduces said caries risk in a subject having a high caries risk.

### Background to the Invention

Dental caries (tooth decay) is the most prevalent chronic disease in the world. Data from the World Health Organization indicate that 80% of the human population suffers or has suffered from it, and it affects to over 50% of the population at school age (Petersen 2004). Dental caries is caused by the acid produced by microorganisms inhabiting the oral cavity, as a consequence of the fermentation of dietary sugars. This lowers the pH on the tooth surface under a certain threshold, below which the enamel demineralizes, initiating a caries lesion (Takahashi and Nyvad 2011). Once the lesion is cavitated, it is irreversible and the damage can only be restored by clinical intervention, for instance a filling or a tooth implant.

Although cavities are caused by microorganisms, dental caries is a multi-factorial disease (Keyes and Jordan 1963). Apart from the microbiology, both human-related factors such as immune competence, enamel strength, tooth shape or saliva buffering effect, and external, environmental factors such as diet, oral hygiene or fluoride exposure have a direct impact on tooth decay rates.

Despite its high prevalence and its direct and indirect impact on human health, there are still no effective diagnostic tools to predict dental caries and therefore dedicate the appropriate personalized measures to prevent the disease. A large effort has been dedicated to study bacterial composition in the oral cavity (Aas *et al.* 2005, Aas *et al.* 2008, Crielaard *et al.* 2001, Belda-Ferre *et al.* 2012), with the aim of developing tests that could relate the presence of acidogenic organisms to caries risk. This has been performed by direct culturing of specific bacteria (Plonka *et al.* 2012) or by DNA-based chips that would identify the presence of potentially pathogenic species (Olson *et al.* 2011). The former approach has been clinically applied for years, and includes diagnostic kits of caries risk assessment, focused on culturing the acidogenic mutans streptococci and lactobacilli. However, the bacterial counts of these two bacteria, which have traditionally been considered the main etiological agents of dental caries, have proven to provide limited diagnostic value. This can be due to the fact that *Streptococcus mutans* accounts for less than 1% of the total bacterial community in cavities (Gross *et al.* 2012), and that lactobacilli are only found in dentin cavities and not in initial, enamel caries lesions, indicating that this species is not involved in caries initiation (Shah *et al.* 2011). Furthermore, the bacterial community within cavities is variable among individuals and cavities (Simon-Soro *et al.* 2013), hampering the identification of a unique list of pathogenic organisms with predictive value of this polymicrobial disease. In addition, most bacteria-based tests utilize saliva as sample (Yoshizawa *et al.* 2013, Plonka *et al.* 2012), and it has been shown that saliva is not representative of the microbial community at the disease site (Simon-Soro *et al.* 2013).

Saliva is a watery substance secreted by the salivary glands into the oral (buccal) cavity of animals. Human saliva is 99.5% water, and the remaining 0.5% comprises immune-protective (*e.g.* antibacterial and anti-viral) compounds, electrolytes (*e.g.* acidity regulating compounds or buffers), compounds preventing or assisting microbial adhesion or dental plaque formation (*e.g.* mucus, glycoproteins), and enzymes. The enzymes found in saliva are not only essential in beginning digestion of dietary starches and fats, but also play a role in protecting teeth from bacterial decay by breaking down food particles entrapped within dental crevices.

Although caries risk tests based on saliva microbial composition have not been successful to predict tooth decay, saliva is a powerful sample to measure overall body health. It is the most easily available and accessible body fluid, and markers expressed in saliva can be used for diagnosis and patient follow-up of different diseases including cancer, diabetes, hereditary disorders and infections (Schafer *et al.* 2014). Given that saliva contains many molecules that can directly or indirectly influence oral microorganisms and their potential cariogenic effect, measuring the levels of appropriate salivary compounds may provide information to predict caries risk (Guo and Shi 2013).

It is the problem of the present invention to provide an improved process for the prediction of caries risk in a patient, wherein said caries risk is evaluated on the basis of salivary composition analysis. In addition to predicting the caries tendency of an individual, it is a further problem of the present invention to identify the underlying reason for the disease propensity in order to facilitate the design of person-specific preventive treatments or therapies that reduce caries risk. These preventive treatments or therapies are intended to compensate for the anomalous salivary composition, restoring the features of the healthy oral cavity such as the immune-protective, microbial adhesion or buffering features of saliva.

**Brief Description of the Invention**

The present invention relates to a method for predicting caries risk in a subject, characterized in that said method comprises the following steps:

(a) determining the concentration in saliva from said subject of

i) at least one compound selected from salivary components of the immune system; and
ii) at least one compound selected from salivary components involved in microbial adhesion to a surface of the buccal cavity; and
iii) at least one compound selected from salivary components involved in
metabolism in the buccal cavity or in regulating the pH of the buccal cavity;

(b) comparing a response variable with a corresponding reference response variable in a population of subjects without caries or comparing a response variable with a corresponding reference response variable in a population of subjects with caries, wherein a response variable is calculated from more than one concentration determined in step (a); and
(c) determining the caries risk based on the degree of difference in response variable compared in step (b), wherein

iv) a greater degree of difference between the response variable of the subject and the corresponding reference response variable in a population of subjects without caries, or
v) a smaller degree of difference between the response variable of the subject and the corresponding reference response variable in a population of subjects with caries,

is indicative of higher caries risk in said subject.

In a preferred embodiment, the method of the present invention further comprises prescribing a plan of care to a subject determined to have a high caries risk in step (c), wherein said plan of care comprises

(d) comparing at least one concentration used to calculate the response variable compared in step (b) with a corresponding reference concentration in a population of subjects without caries, or comparing at least one concentration used to calculate the response variable compared in step (b) with a corresponding reference concentration in a population of subjects with caries; and
(e) determining the imbalance in said concentration based on the degree of difference in concentration compared in step (d), wherein

(vi) a greater degree of difference between the concentration of the subject and the corresponding reference concentration in a population of subjects without caries, or
(vii) a smaller degree of difference between the concentration of the subject and the corresponding reference concentration in a population of subjects with caries,

is indicative of higher imbalance in said concentration; and
(f) modulating

(viii) the immune system, when a compound whose concentration is determined to have a high imbalance in step (e) is a compound selected from salivary components of the immune system;
(ix) microbial adhesion to a surface of the buccal cavity, when a compound whose concentration is determined to have a high imbalance in step (e) is a compound selected from salivary components involved in microbial adhesion to a surface of the buccal cavity; and/or
(x) metabolism in the buccal cavity or the pH of the buccal cavity, when a compound whose concentration is determined to have a high imbalance in step (e) is a compound selected from salivary components involved in metabolism in the buccal cavity or in regulating the pH of the buccal cavity.

In addition, the present invention relates to a kit for predicting caries risk in a subject characterized in that said kit comprises:

(a) means for determining the concentration in saliva from said subject of

> i) at least one compound selected from salivary components of the immune system; and
> ii) at least one compound selected from salivary components involved in microbial adhesion to a surface of the buccal cavity; and
> iii) at least one compound selected from salivary components involved in metabolism in the buccal cavity or in regulating the pH of the buccal cavity;

(b) instructions for comparing a response variable with a corresponding reference response variable in a population of subjects without caries or comparing a response variable with a corresponding reference response variable in a population of subjects with caries, wherein a response variable is calculated from more than one concentration determined in step (a); and

(c) instructions for determining the caries risk based on the degree of difference in response variable compared in step (b), wherein

> iv) a greater degree of difference between the response variable of the subject and the corresponding reference response variable in a population of subjects without caries, or
> v) a smaller degree of difference between the response variable of the subject and the corresponding reference response variable in a population of subjects with caries,

is indicative of higher caries risk in said subject.

In a preferred embodiment, the kit of the present invention further comprises instructions for prescribing a plan of care to a subject determined to have a high caries risk in step (c), wherein said plan of care comprises

> (d) comparing at least one concentration used to calculate the response variable compared in step (b) with a corresponding reference concentration in a population of subjects without caries, or comparing at least one concentration used to calculate the response variable compared in step (b) with a corresponding reference concentration in a population of subjects with caries; and
> (e) determining the imbalance in said concentration based on the degree of difference in concentration compared in step (d), wherein

>> (vi) a greater degree of difference between the concentration of the subject and the corresponding reference concentration in a population of subjects without caries, or
>> (vii) a smaller degree of difference between the concentration of the subject and the corresponding reference concentration in a population of subjects with caries,

> is indicative of higher imbalance in said concentration; and
> (f) modulating

>> (viii) the immune system, when a compound whose concentration is determined to have a high imbalance in step (e) is a compound selected from salivary components of the immune system;
>> (ix) microbial adhesion to a surface of the buccal cavity, when a compound whose concentration is determined to have a high imbalance in step (e) is a compound selected from salivary components involved in microbial adhesion to a surface of the buccal cavity; and/or
>> (x) metabolism in the buccal cavity or the pH of the buccal cavity, when a compound whose concentration is determined to have a high imbalance in step (e) is a compound selected from salivary components involved in metabolism in the buccal cavity or in regulating the pH of the buccal cavity.

Moreover, the present invention additionally relates to use of the method according to the present invention described herein or the kit according to the present invention described herein, in predicting caries risk in a subject.

**Brief Description of the Figures**

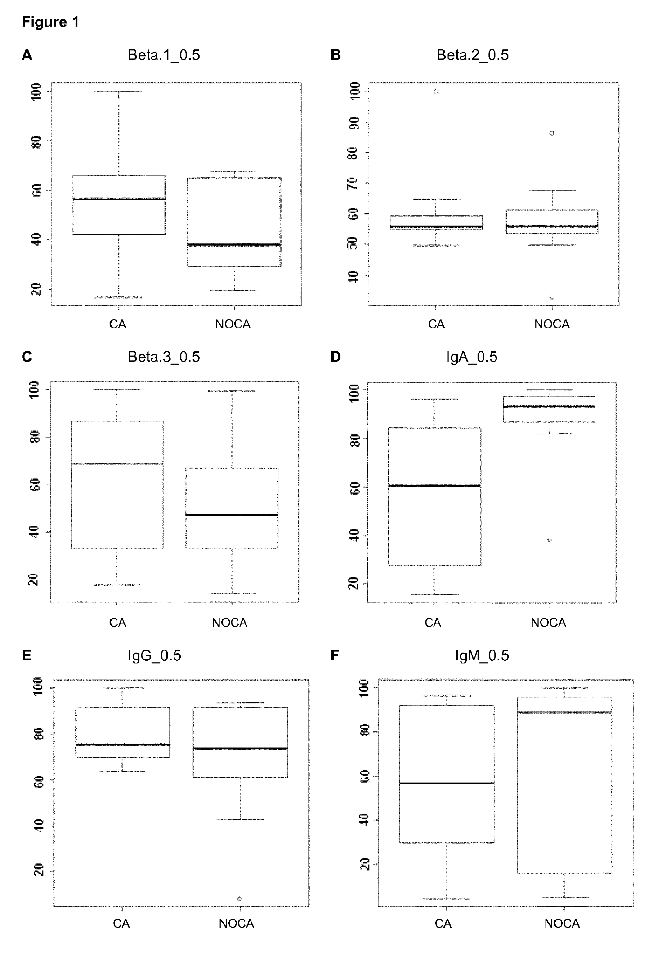

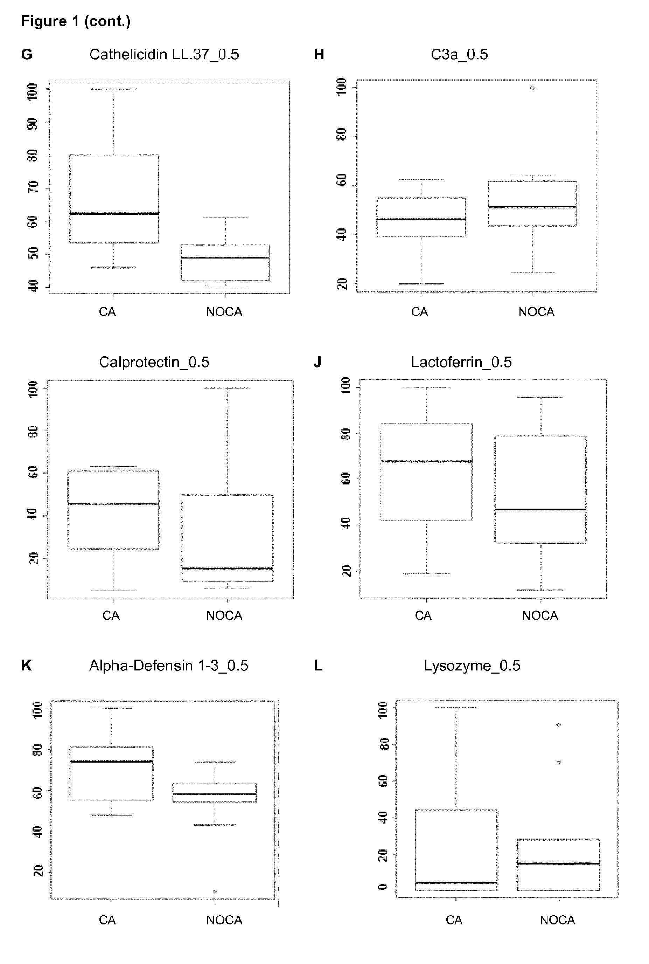

> **Figure 1.** Box-and-whisker plots showing the means, standard variations and upper/lower quartiles of the normalized concentrations of salivary immune system components (normalized as percentages of the maximum concentration obtained of each component) for samples of saliva collected from populations of caries-free (NOCA) and caries-bearing (CA) patients 0.5 hours after brushing teeth (wherein ° = outlier), wherein said salivary immune system components are **A** Beta-Defensin 1 (ng/mL), **B** Beta-Defensin 2 (pg/mL), **C** Beta-Defensin 3 (ng/mL), **D** IgA (mg/dL), **E** IgG (mg/dL), **F** IgM (mg/dL), **G** Cathelicidin LL-37 (ng/mL), **H** C3a ($\mu$g/mL), I Calprotectin ($\mu$g/mL), J Lactoferrin (mg/L), **K** Alpha-Defensin 1-3 (ng/mL) and L Lysozyme (ng/mL).

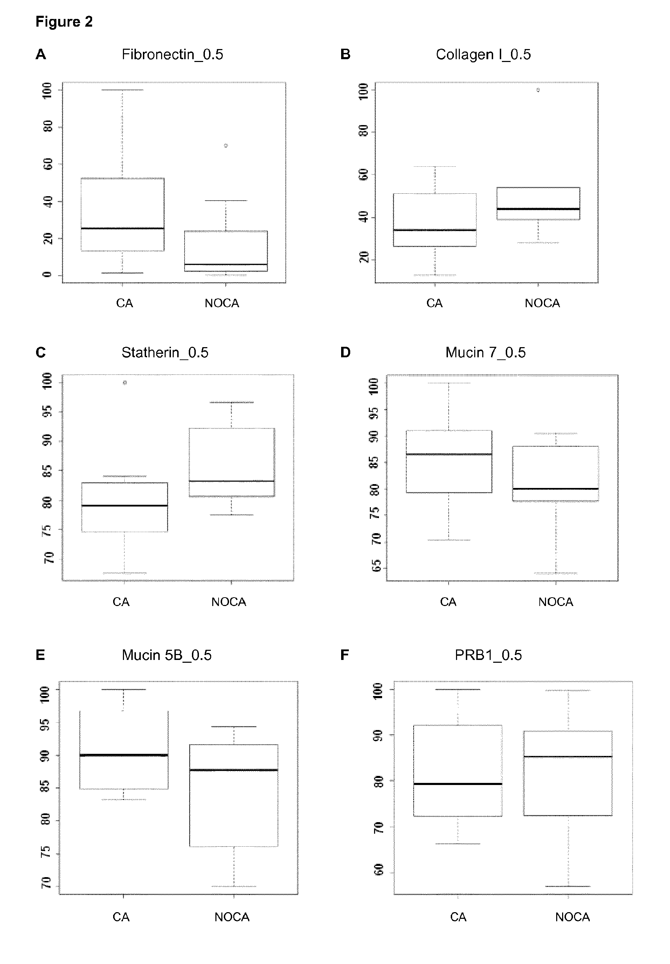

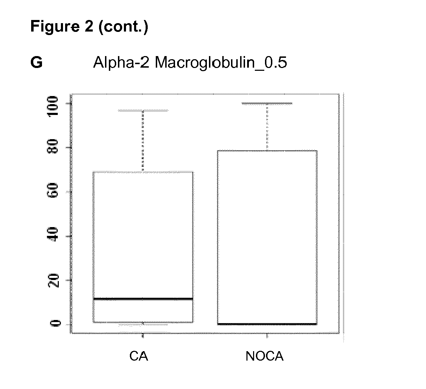

**Figure 2.** Box-and-whisker plots showing the means, standard variations and upper/lower quartiles of the normalized concentrations of salivary adhesion components (normalized as percentages of the maximum concentration obtained of each component) for samples of saliva collected from populations of caries-free (NOCA) and caries-bearing (CA) patients 0.5 hours after brushing teeth (wherein ° = outlier), wherein said salivary adhesion components are **A** Fibronectin ($\mu$g/mL), **B** Collagen I ($\mu$g/mL), **C** Statherin (ng/mL), **D** Mucin 7 (ng/mL), **E** Mucin 5B (ng/mL), **F** PRB1 (ng/mL) and **G** Alpha-2 Macroglobulin ($\mu$g/mL).

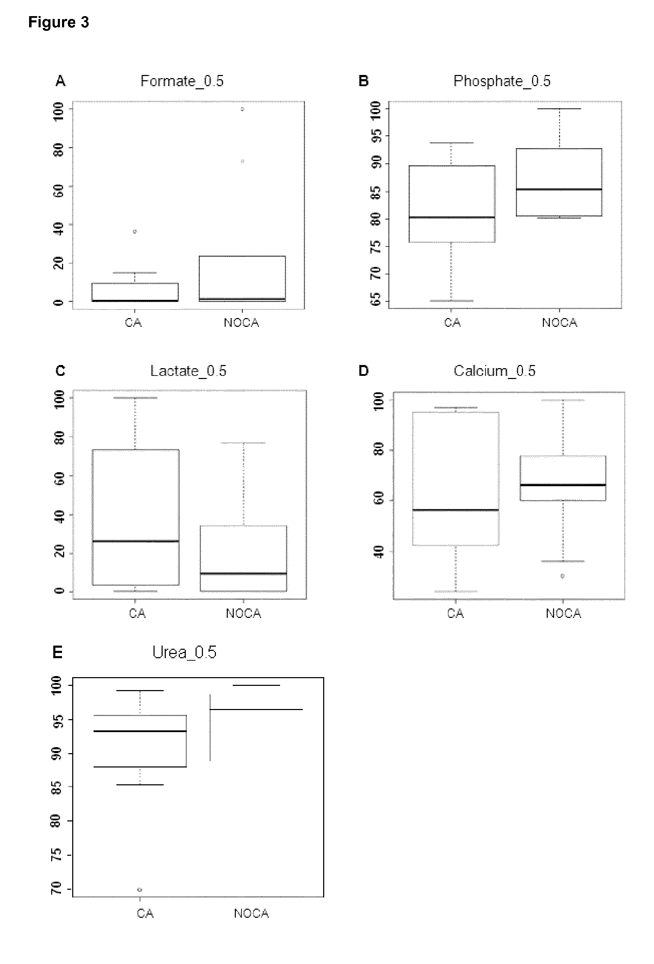

**Figure 3.** Box-and-whisker plots showing the means, standard variations and upper/lower quartiles of the normalized concentrations of salivary acidity-regulating components (normalized as percentages of the maximum concentration obtained of each component) for samples of saliva collected from populations of caries-free (NOCA) and caries-bearing (CA) patients 0.5 hours after brushing teeth (wherein ° = outlier), wherein said salivary acidity-regulating components are **A** Formate ($\mu$mol/mL), **B** Phosphate ($\mu$mol/mL), **C** Lactate ($\mu$mol/mL), **D** Calcium (mg/mL) and **E** Urea ($\mu$mol/mL).

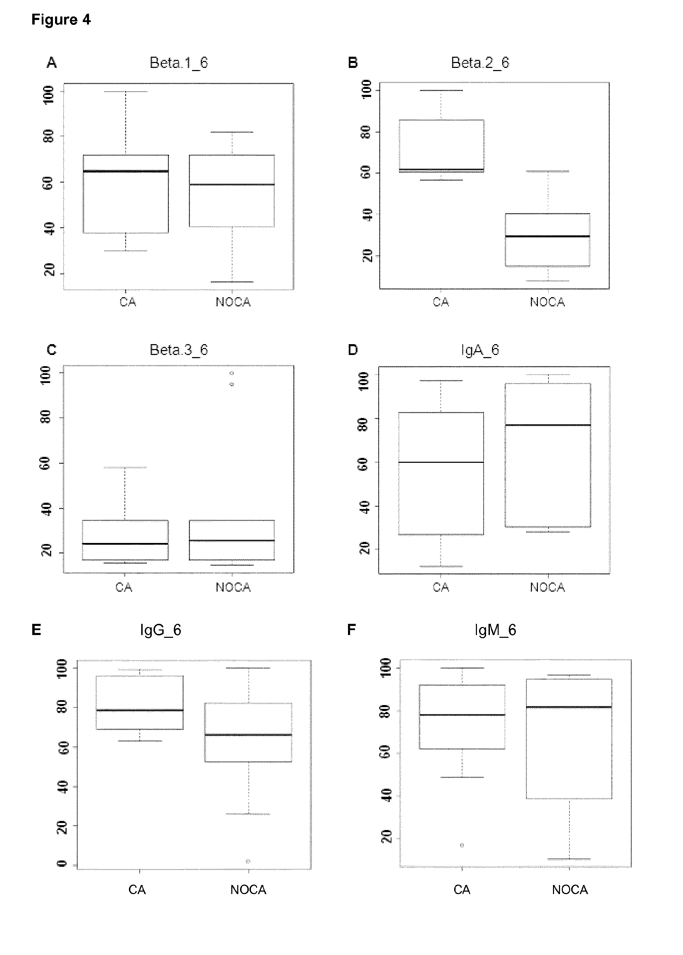

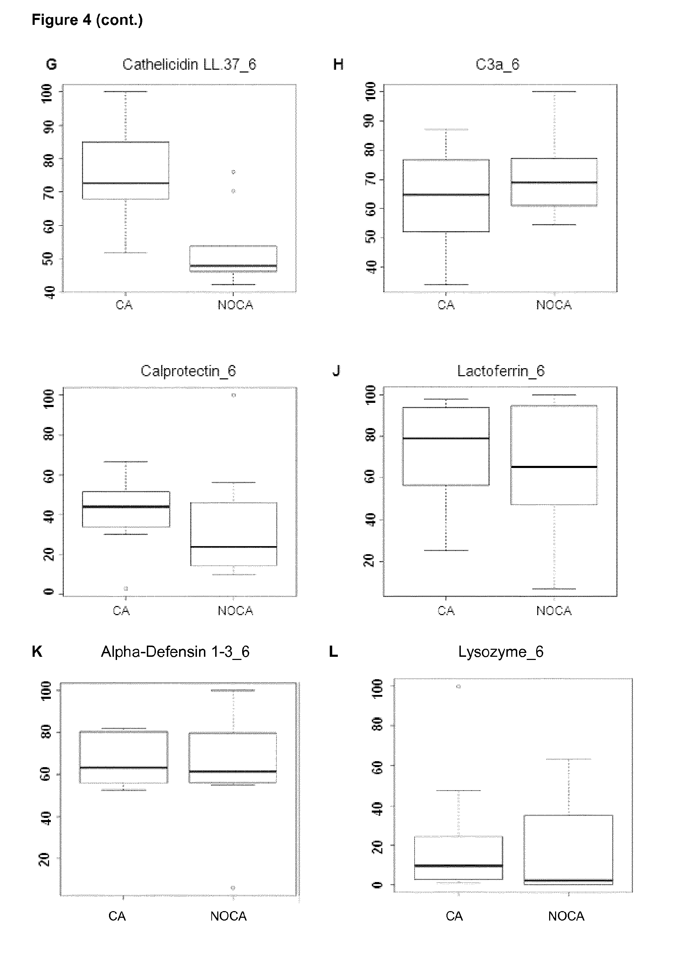

**Figure 4.** Box-and-whisker plots showing the means, standard variations and upper/lower quartiles of the normalized concentrations of salivary immune system components (normalized as percentages of the maximum concentration obtained of each component) for samples of saliva collected from populations of caries-free (NOCA) and caries-bearing (CA) patients 6 hours after brushing teeth (wherein ° = outlier), wherein said salivary immune system components are **A** Beta-Defensin 1 (ng/mL), **B** Beta-Defensin 2 (pg/mL), **C** Beta-Defensin 3 (ng/mL), **D** IgA (mg/dL), **E** IgG (mg/dL), **F** IgM (mg/dL), **G** Cathelicidin LL-37 (ng/mL), **H** C3a ($\mu$g/mL), I Calprotectin ($\mu$g/mL), **J** Lactoferrin (mg/L), **K** Alpha-Defensin 1-3 (ng/mL) and **L** Lysozyme (ng/mL).

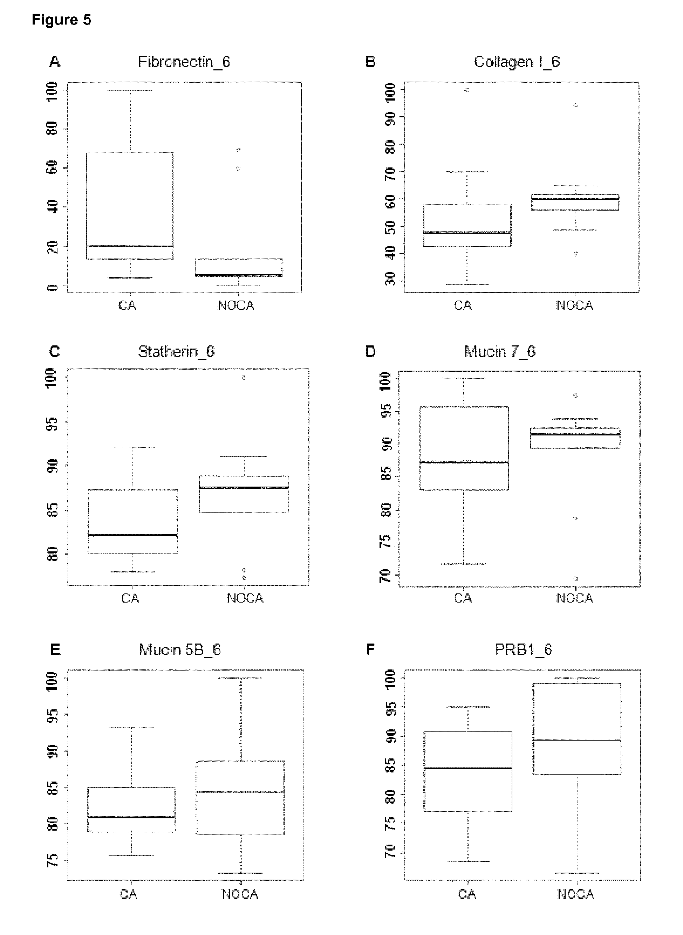

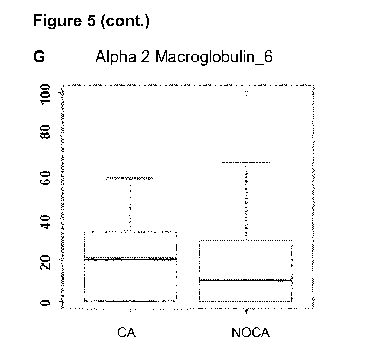

**Figure 5.** Box-and-whisker plots showing the means, standard variations and upper/lower quartiles of the normalized concentrations of salivary adhesion components (normalized as percentages of the maximum concentration obtained of each component) for samples of saliva collected from populations of caries-free (NOCA) and caries-bearing (CA) patients 6 hours after brushing teeth (wherein ° = outlier), wherein said salivary adhesion components are **A** Fibronectin ($\mu$g/mL), **B** Collagen I ($\mu$g/mL), **C** Statherin (ng/mL), **D** Mucin 7 (ng/mL), **E** Mucin 5B (ng/mL), **F** PRB1 (ng/mL) and **G** Alpha-2 Macroglobulin ($\mu$g/mL).

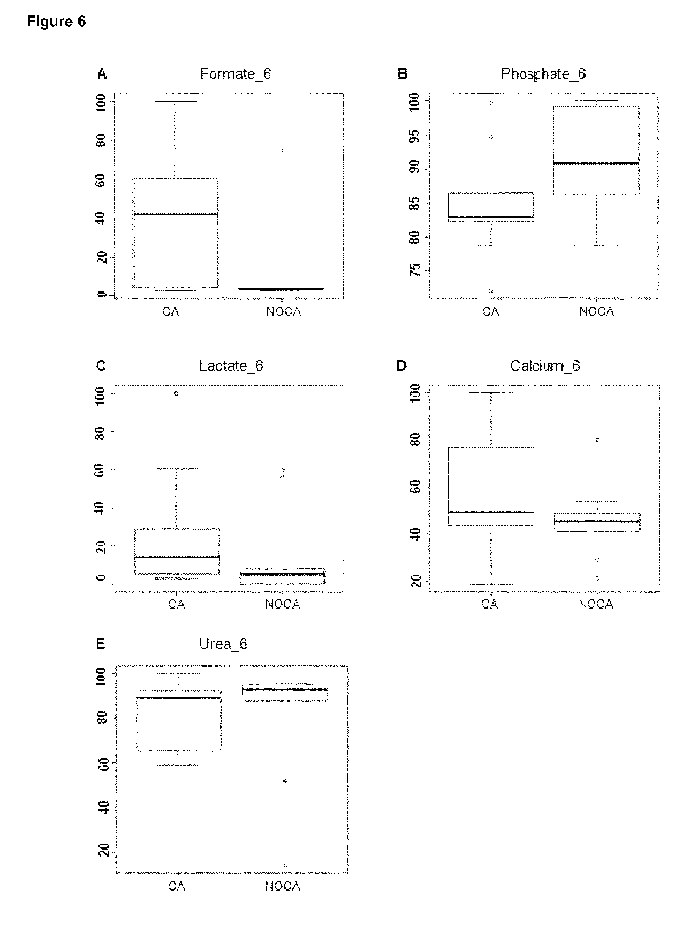

**Figure 6.** Box-and-whisker plots showing the means, standard variations and upper/lower quartiles of the normalized concentrations of salivary acidity-regulating components (normalized as percentages of the maximum concentration obtained of each component) for samples of saliva collected from populations of caries-free (NOCA) and caries-bearing (CA) patients 6 hours after brushing teeth (wherein ° = outlier), wherein said salivary acidity-regulating components are **A** Formate ($\mu$mol/mL), **B** Phosphate ($\mu$mol/mL), **C** Lactate ($\mu$mol/mL), **D** Calcium (mg/mL) and **E** Urea ($\mu$mol/mL).

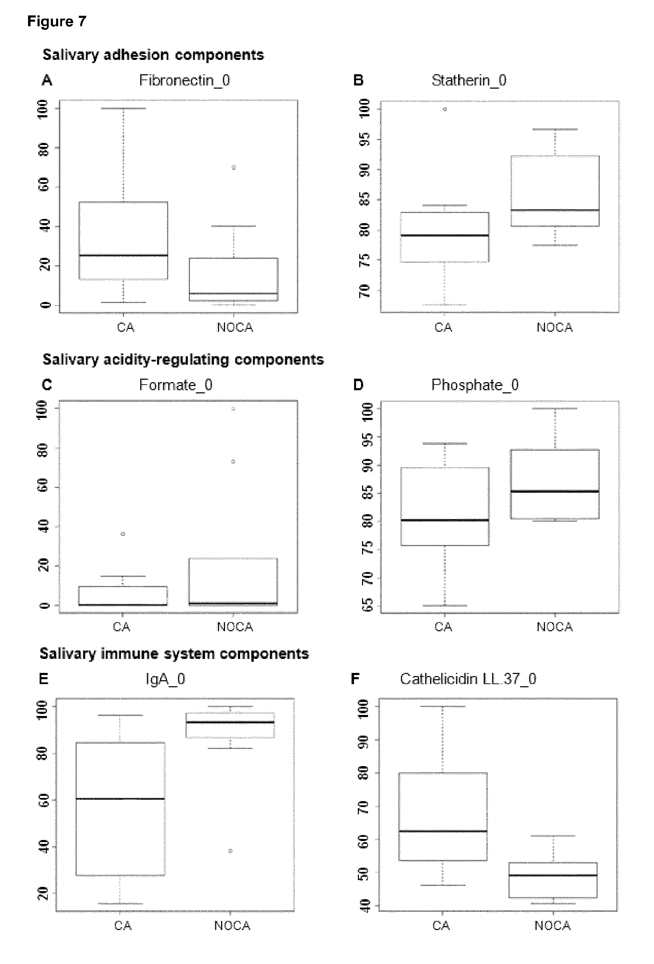

**Figure 7.** Box-and-whisker plots showing the means, standard variations and upper/lower quartiles of the normalized concentrations of salivary immune system, adhesion and acidity-regulating components (normalized as percentages of the maximum concentration obtained of each component) for samples of saliva collected from populations of caries-free (NOCA) and caries-bearing (CA) patients 0.5 hours after brushing teeth (wherein ° = outlier), wherein said salivary adhesion components are **A** Fibronectin ($\mu$g/mL) and **B** Statherin (ng/mL), said salivary acidity-regulating components are **C** Formate ($\mu$mol/mL) and **D** Phosphate ($\mu$mol/mL), and said salivary immune system components are **E** IgA (mg/dL) and **F** Cathelicidin LL-37 (ng/mL).

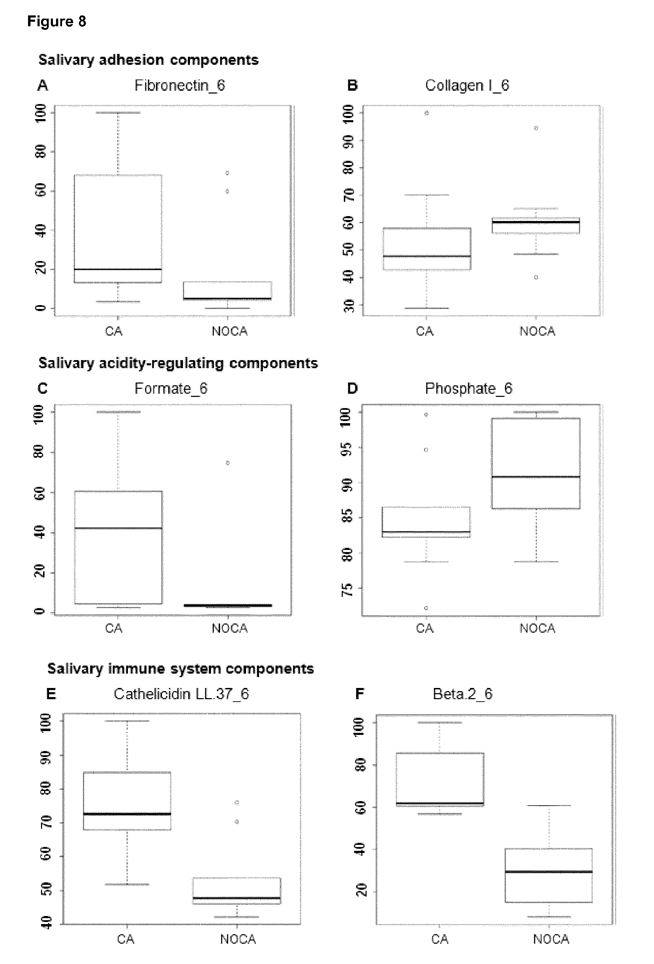

**Figure 8.** Box-and-whisker plots showing the means, standard variations and upper/lower quartiles of the normalized concentrations of salivary immune system, adhesion and acidity-regulating components (normalized as percentages of the maximum concentration obtained of each component) for samples of saliva collected from populations of caries-free (NOCA) and caries-bearing (CA) patients 6 hours after brushing teeth (wherein ° = outlier), wherein said salivary adhesion components are **A** Fibronectin ($\mu$g/mL) and **B** Collagen I ($\mu$g/mL), said salivary acidity-regulating components are **C** Formate ($\mu$mol/mL) and **D** Phosphate ($\mu$mol/mL), and said salivary immune system components are **E** Cathelicidin LL-37 (ng/mL) and **F** Beta-Defensin 2 (pg/mL).

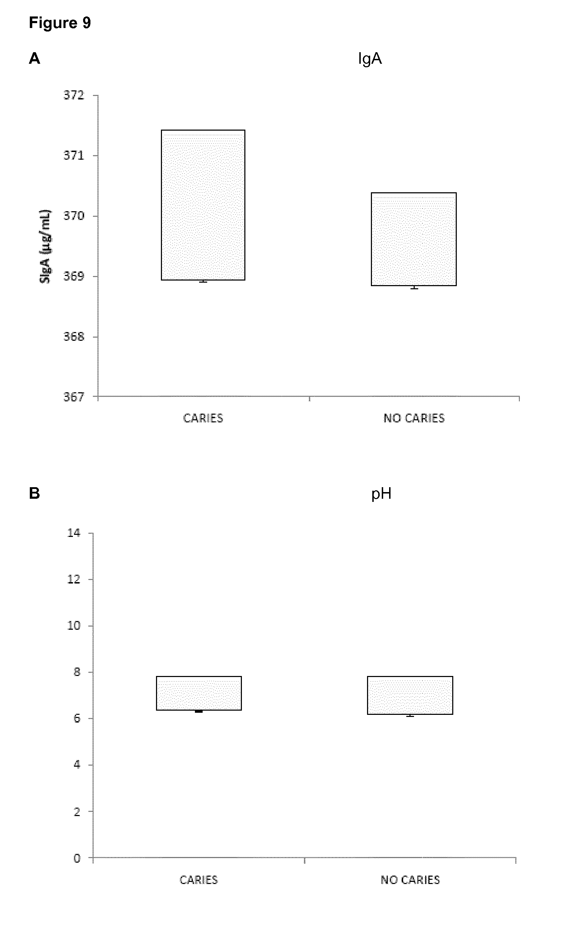

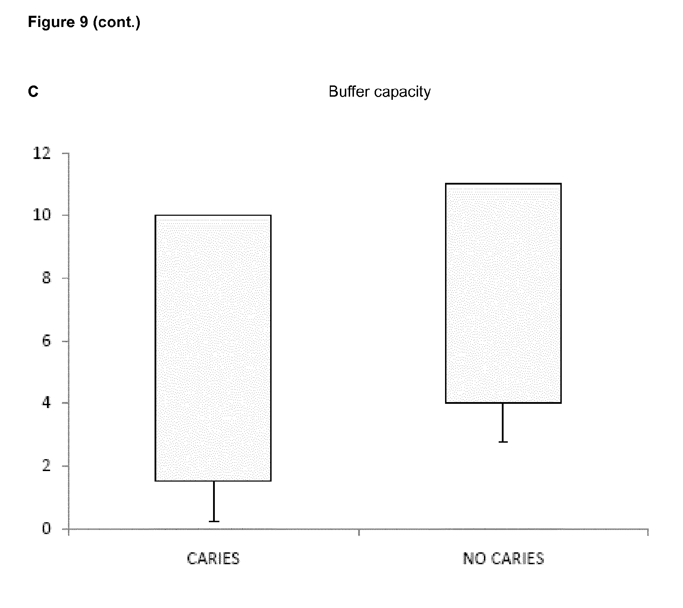

**Figure 9.** Box-and-whisker plots showing the means, standard variations and upper/lower quartiles of **A** concentrations of IgA (measured using ELISA), **B** pH or **C** buffer capacity, determined according to protocols employed in commercially-available kits for detecting caries risk in patients, in samples of saliva collected from populations of caries-free (NOCA) and caries-bearing (CA) patients.

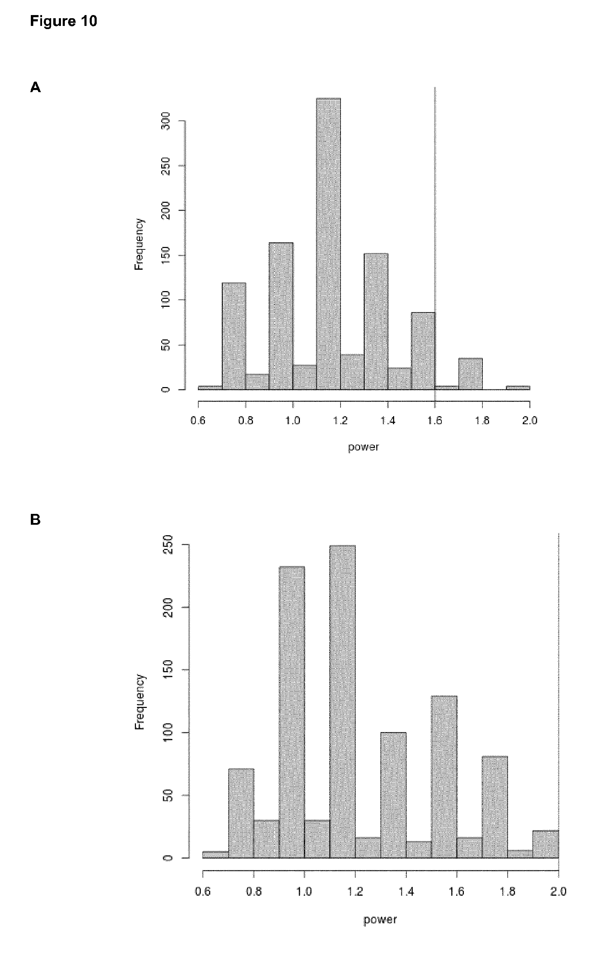

**Figure 10.** Statistical classification power of 1000 combinations of concentrations of six components randomly selected from the 24 components measured in saliva, including the combination of concentrations of the six components employed in the present invention, for samples taken at **A** 0.5 hours and **B** 6 hours after brushing teeth. Median classification power of the 6 randomly selected compounds = 1.2 at both 0.5 and 6 hours, whereas the median classification power of the present invention (vertical line) = 1.6 and 2.0 at 0.5 and 6 hours after brushing teeth, respectively. Power = [proportion of correct classification of caries individuals (CA)] + [proportion of correct classification of caries-free individuals (NOCA)], wherein said proportions were estimated using a 50-fold cross validation approach.

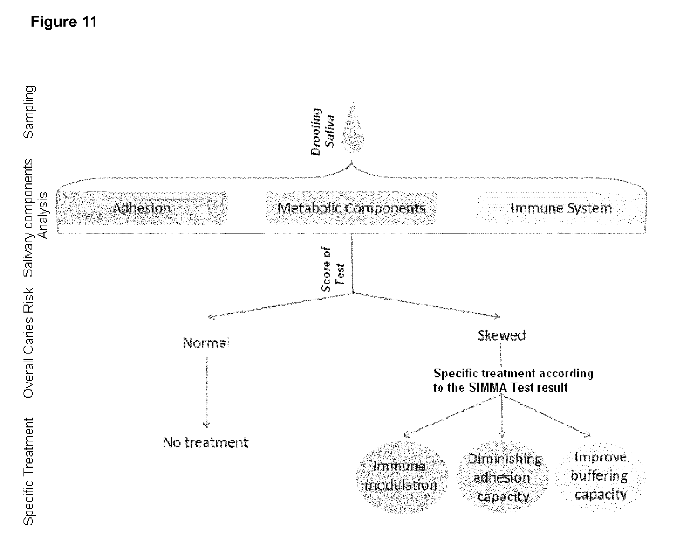

**Figure 11.** Flow chart of the Salivary Immune and Metabolic Marker Analysis (SIMMA) test, showing its three stages of (1) sampling (collection of drooled saliva), (2) salivary components analysis (measurement of salivary immune system, adhesion and acidity-regulating components concentrations) and (3) overall caries risk assessment (determining whether a given patient score of test is normal or skewed), the result of which is used, where relevant, to decide on a specific treatment selected from immune modulation, diminishing adhesion capacity and/or improving buffering capacity.

**Detailed Description of the Invention**

The present invention relates to a method for predicting caries risk in a subject.

Said method comprises the following steps:

(a) determining the concentration in saliva from said subject of

i) at least one compound selected from salivary components of the immune system; and
ii) at least one compound selected from salivary components involved in microbial adhesion to a surface of the buccal cavity; and
iii) at least one compound selected from salivary components involved in metabolism in the buccal cavity or in regulating the pH of the buccal cavity;

(b) comparing a response variable with a corresponding reference response variable in a population of subjects without caries or comparing a response variable with a corresponding reference response variable in a population of subjects with caries, wherein a response variable is calculated from more than one concentration determined in step (a); and
(c) determining the caries risk based on the degree of difference in response variable compared in step (b), wherein

iv) a greater degree of difference between the response variable of the subject and the corresponding reference response variable in a population of subjects without caries, or
v) a smaller degree of difference between the response variable of the subject and the corresponding reference response variable in a population of subjects with caries,
is indicative of higher caries risk in said subject.

Saliva is the liquid secreted by the salivary glands into the buccal cavity of a subject. For the purposes of the present invention, saliva is the liquid obtained from the buccal cavity of a subject, preferably the liquid obtained from the buccal cavity of a subject by drooling (*i.e.* as non-stimulated saliva).

Saliva comprises a mixture of molecules (components) that can be related to caries propensity. Salivary components that can be related to caries propensity include salivary components of the immune system, salivary components involved in microbial adhesion to a surface of the buccal cavity, and salivary components involved in metabolism in the buccal cavity or in regulating the pH of the buccal cavity. Specifically, the salivary components related to the immune system include immunoglobulins, antimicrobial peptides and proteins of the complement system. In addition, the salivary components involved in microbial adhesion to a surface of the buccal cavity are those that are related to the adhesion capacity of microorganisms, and include structural components of saliva that microorganisms use as targets for sticking to teeth and for forming dental plaque. Moreover, salivary components involved in metabolism in the buccal cavity or in regulating the pH of the buccal cavity are components that are related to the acidity of saliva, and include enzymes that metabolize sugars, acidic compounds that are produced as a consequence of sugar fermentation and those salivary components that can act as acid neutralizers.

Thus, the method of the present invention comprises a step (a) of determining the concentration in saliva from a subject of

i) at least one compound selected from salivary components of the immune system; and

ii) at least one compound selected from salivary components involved in microbial adhesion to a surface of the buccal cavity; and

iii) at least one compound selected from salivary components involved in metabolism in the buccal cavity or in regulating the pH of the buccal cavity;

In a preferred embodiment, the method of the present invention comprises a step (a) of determining the concentration in saliva from a subject wherein

- the at least one compound selected from salivary components of the immune system is at least one compound selected from immunoglobulins, an antimicrobial peptide and/or a protein of the complement system;
- the at least one compound selected from salivary components involved in microbial adhesion to a surface of the buccal cavity is at least one compound selected from salivary components to which microorganisms adhere; and
- the at least one compound selected from salivary components involved in metabolism in the buccal cavity or in regulating the pH of the buccal cavity is at least one compound selected from a buffering agent, an agent that neutralizes acids, an enzyme that metabolizes at least one sugar and/or a compound produced during fermentation.

In more preferred embodiment, the method of the present invention comprises a step (a) of determining the concentration in saliva from a subject wherein

- the at least one compound selected from salivary components of the immune system is at least one compound selected from the list comprising Cathelicidin LL-37, IgA, Alpha-Defensin 1-3, IgM, Beta-Defensin 1, Lactoferrin, C3a, Calprotectin, Beta-Defensin 3, IgG, Beta-Defensin 2 and Lysozyme;
- the at least one compound selected from salivary components involved in microbial adhesion to a surface of the buccal cavity is at least one compound selected from the list comprising Statherin, Fibronectin, Mucin 5B, Mucin 7, Collagen I, Alpha-2 Macroglobulin and PRB1; and
- the at least one compound selected from salivary components involved in metabolism in the buccal cavity or in regulating the pH of the buccal cavity is at least one compound selected from the list comprising Phosphate, Lactate, Urea, Calcium and Formate.

In yet more preferred embodiment, the method of the present invention comprises a step (a) of determining the concentration in saliva from a subject wherein

- the at least one compound selected from salivary components of the immune system is at least one compound selected from the list comprising Cathelicidin LL-37, IgA and Beta-Defensin 2;
- the at least one compound selected from salivary components involved in microbial adhesion to a surface of the buccal cavity is at least one compound selected from the list comprising Statherin, Fibronectin and Collagen I; and
- the at least one compound selected from salivary components involved in metabolism in the buccal cavity or in regulating the pH of the buccal cavity is at least one compound selected from the list comprising Phosphate, Lactate and Formate.

In furthermore preferred embodiment, the method of the present invention comprises a step (a) of determining the concentration in saliva from a subject wherein

- the at least one compound selected from salivary components of the immune system is at least one compound selected from the list comprising Cathelicidin LL-37 and IgA;
- the at least one compound selected from salivary components involved in microbial adhesion to a surface of the buccal cavity is at least one compound selected from the list comprising Statherin and Fibronectin; and
- the at least one compound selected from salivary components involved in metabolism in the buccal cavity or in regulating the pH of the buccal cavity is at least one compound selected from the list comprising Phosphate and Lactate, and the concentration in saliva is determined between 0 and 2 hours after swilling with a sucrose solution and/or brushing teeth, or wherein
- the at least one compound selected from salivary components of the immune system is at least one compound selected from the list comprising Cathelicidin LL-37 and Beta-Defensin 2;

- the at least one compound selected from salivary components involved in microbial adhesion to a surface of the buccal cavity is at least one compound selected from the list comprising Fibronectin and Collagen I; and
- the at least one compound selected from salivary components involved in metabolism in the buccal cavity or in regulating the pH of the buccal cavity is at least one compound selected from the list comprising Phosphate and Formate, and

the concentration in saliva is determined between 2 and 24 hours after swilling with a sucrose solution and/or brushing teeth.

In the present invention, a subject and each subject in the population of subjects without caries is an animal subject, preferably a mammalian subject. More preferably, a subject and each subject in the population of subjects without caries is a hominine subject, a canine subject, a feline subject, a bovine subject, an ovine subject, a porcine subject, an equine subject, a camelline subject, a caprine subject or a cervine subject. Furthermore preferably, said subject and each subject in the population of subjects without caries is a human, dog, cow, horse or camel, most preferably a human. Said subject and each subject in the population of subjects without caries may be a subject with deciduous teeth or permanent teeth, *i.e.* said subject may be from any age group, whether it be a child, a teenager or an adult. Preferably, the subject and the population of subjects without caries belong to the same species, sex, age and/or race.

In the present invention, the step (a) of determining the concentration in saliva from the subject of i) at least one compound selected from salivary components of the immune system; ii) at least one compound selected from salivary components involved in microbial adhesion to a surface of the buccal cavity; and iii) at least one compound selected from salivary components involved in metabolism in the buccal cavity or in regulating the pH of the buccal cavity; involves measuring the level of said compounds in a sample of saliva obtained from said subject. Determining the concentration of said compounds is preferably performed by standard analytical chemical techniques or instruments (means), selected from spectrophotometric, mass spectrometric, gravimetric, volumetric, calorimetric, electrochemical, microelectromechanical (lab-on-a-chip) and immunohistochemical techniques or instruments, or combinations thereof, preferably spectrophotometric, mass spectrometric, microelectromechanical and immunohistochemical techniques or instruments, or combinations thereof. Examples of techniques or instruments for detecting proteins which employ spectrophotometric methods include, in particular, Enzyme-Linked ImmunoSorbent Assay (ELISA tests), Microsphere-Based Multiplex Immunoassays, the 280 nm absorbance method, 2D-Quant kit, Bradford protein assay, Lowry, non-interfering protein assay (G-Biosciences), BCA, Biuret, fluoraldehyde protein/peptide assay, ESL protein assay, Lowry-Peterson assay, butterfly assay and 660 nm protein assay.

In the present invention, the saliva is a sample of saliva obtained from the buccal cavity of a subject. Preferably the saliva is obtained by ejection from the buccal cavity by passive means such as drooling, or active means, such as by syringing, capillary action or absorption from the buccal cavity. More preferably, the saliva is obtained by drooling. The saliva is not obtained from the buccal cavity by spitting, nor directly from the salivary glands.

In a preferred embodiment of the present invention, the saliva is obtained at the same time in a subject as in the population of subjects without caries. In a more preferred embodiment, the saliva used for determining the concentration of at least one compound selected from salivary components of the immune system, and of at least one compound selected from salivary components involved in microbial adhesion to a surface of the buccal cavity is obtained at a time (a1) of duration z after brushing teeth, and/or at a time (b1) of a duration y after brushing teeth; and the saliva used for determining the concentration of at least one compound selected from salivary components involved in metabolism in the buccal cavity or in regulating the pH of the buccal cavity is obtained:

i) at a time (a2) coincident with the aforementioned time (a1) and a time of a duration x after swilling with a sucrose solution, and/or at a time (b2) coincident with the aforementioned time (b1) and a time of said duration x after swilling with a second sucrose solution, wherein swilling with a sucrose solution is performed after brushing teeth; or
ii) at a time (a3) coincident with a time of said duration z prior to the aforementioned time (a1) (*i.e.* immediately prior to brushing teeth) and a time of said duration x after swilling with a sucrose solution, and/or at a time (b3) coincident with a time of said duration z prior to the aforementioned time (b1) and a time of said duration x after swilling with a sucrose solution; or
iii) at a time (c2) of said duration x after swilling with a sucrose solution, wherein swilling with a sucrose solution is performed immediately after obtaining the saliva sample obtained at time (a1) and/or time (b1),

wherein duration z is between 0 and 2 hours, duration y is between 2 and 24 hours and duration x is between 0 and 20 minutes. More preferably, duration z is between 15 minutes and 45 minutes, duration y is between 4 and 12 hours and duration x is between 5 and 15 minutes. Yet more preferably, duration z is between 20 and 40 minutes, duration y is between 4 and 8 hours and duration x is 10 minutes. Furthermore preferably, duration z is 30 minutes, duration y is 6 hours and duration x is 10 minutes.

In an especially preferred embodiment, the saliva is obtained at a time (a) coincident with between 0 and 2 hours after brushing teeth and between 0 and 20 minutes after swilling with a sucrose solution, and/or at a time (b) coincident with between 2 and 24 hours after brushing teeth and between 0 and 20 minutes after swilling with a second sucrose solution, wherein each step of swilling with a sucrose solution is performed after brushing teeth. More preferably, the saliva is obtained at a time (a) coincident with between 15 minutes and 45 minutes after brushing teeth and between 5 and 15 minutes after swilling with a sucrose solution, and/or at a time (b) coincident with between 4 and 12 hours after brushing teeth and between 5 and 15 minutes after swilling with a second sucrose solution. Yet more preferably, the saliva is obtained at a time (a) coincident with between 20 minutes and 40 minutes after brushing teeth and 10 minutes after swilling with a sucrose solution, and/or at a time (b) coincident with between 4 and 8 hours after brushing teeth and 10 minutes after swilling with a second sucrose solution. Most preferably, the saliva is obtained at a time (a) coincident with 30 minutes after brushing teeth and 10 minutes after swilling with a sucrose solution, and/or at a time (b) coincident with 6 hours after brushing teeth and 10 minutes after swilling with a second sucrose solution.

All of the aforementioned times may have an error of $\pm$ 2 minutes, more preferably $\pm$ 1 minute. No action other than swallowing or drooling should be taken in the interval between brushing the teeth and taking the first saliva sample, and/or swilling with a sucrose solution and taking the first saliva sample. For the purposes of the invention, the aforementioned times of obtaining saliva correspond with the times at which concentrations of the salivary components are determined, since said samples may be frozen until use or employed directly.

For the purposes of the present invention, swilling comprises contacting at least 75%, more preferably at least 90%, furthermore preferably at least 95% of the dental surfaces of the buccal cavity with a sucrose solution. The sucrose solution is preferably an aqueous solution of sucrose at up to 65 w/v% sucrose (whereby w/v% is the percentage of weight of sucrose by volume of water), more preferably between 5 and 40 w/v% sucrose, furthermore preferably between 10 and 30 w/v%. Swilling more preferably comprises retaining a volume of between 5 and 100 mL, preferably between 10 and 50 mL, of said sucrose solution in contact with at least 75%, more preferably at least 90%, furthermore preferably at least 95% of the dental surfaces of the buccal cavity (without swallowing) for one minute $\pm$ 30 seconds, after which time the sucrose solution is preferably expelled by drooling, not spitting. Swilling may be performed irrespective of whether or not a meal has been eaten between the times (a) and (b), (a1) and (b1), or (a2) or (d2) and (b2) at which a saliva sample is obtained, to guarantee that salivary acidity-regulating components are released as a consequence of sugar fermentation, regardless of the time since last ingested meal.

For the purposes of the present invention, brushing teeth comprises applying physical action to at least 50%, more preferably at least 70%, of the dental surfaces of the buccal cavity, more preferably without chemical action, that is, brushing teeth is performed without toothpaste or equivalent cleaning products. Preferably, brushing teeth comprises applying physical scrubbing action to at least 50%, more preferably at least 70%, of the dental surfaces of the buccal cavity. For the purposes of the invention, brushing teeth comprises actions inherently associated with this action, including brushing using a toothbrush or other dental tool, rinsing with water and spitting.

The method of the present invention additionally comprises steps (b) of comparing a response variable with a corresponding reference response variable in a population of subjects without caries or comparing a response variable with a corresponding reference response variable in a population of subjects with caries, wherein a response variable is calculated from more than one concentration determined in step (a); and (c) of determining the caries risk based on the degree of difference in response variable compared in step (b), wherein iv) a greater degree of difference between the response variable of the subject and the corresponding reference response variable in a population of subjects without caries, or v) a smaller degree of difference between the response variable of the subject and the corresponding reference response variable in a population of subjects with caries, is indicative of higher caries risk in said subject. Thus, in the present invention, steps (b) and (c) are performed using the concentrations of multiple compounds by multivariate analysis. Multivariate analysis involves comparing a response variable with a corresponding reference response variable in a population of subjects without caries, or comparing a response variable with a corresponding reference response variable in a population of subjects with caries, wherein a response variable is calculated from more than one concentration determined in step (a), and using the difference in said at least one response variable to determine the caries risk. A response variable is preferably calculated from more than one concentration determined in step (a) using a model (an equation) which accounts for statistical interaction between concentrations, and is optimized to distinguish between populations of subjects with caries and populations of subjects without caries, wherein said model assumes that the response variable, which contains the status (CA or NOCA) of each individual, follows a binomial distribution with parameter p, and p corresponds to the probability for the response variable of taking value NOCA. Preferably optimization is achieved by selecting that model which is most accurate at distinguishing between populations of subjects with caries and populations of subjects without caries, wherein the accuracy of each model is calculated by means of 50-fold cross-validation and compared with that of a model of independence which only includes linear predictor selection variables and does not take into account any interactions between concentrations. More preferably, multiple models are developed for subjects from different populations according to species, sex, age and/or race.

Comparing the response variable with a corresponding reference response variable in a population of subjects without caries, means evaluating whether there is a difference in response variables between that of said subject and that of said population of healthy subjects. Equally, comparing the response variable with a corresponding reference response variable in a population of subjects with caries, means evaluating whether there is a difference in response variables between that of said subject and that of said population of subjects with caries. In each case, said difference is a numerical difference based on a threshold value, preferably a statistically significant difference. When said difference is such that said response variable determined in step (a) is statistically significantly greater than or statistically significantly less than said corresponding reference response variable in a population of subjects without caries, or is statistically significantly greater than or statistically significantly less than said corresponding reference response variable in a population of subjects without caries, this is indicative of caries risk in said subject, *i.e.* that the subject may be suffering from or susceptible to caries.

Alternatively, the corresponding reference response variable of a population of subjects without caries is based on *(i.e.* corresponds with) the specificity of the method of the invention. As mentioned above, specificity is the ability of the method to correctly identify those subjects who do not have caries from a population of healthy subjects. For example, in the method of the invention the corresponding reference response variable in a population of subjects without caries is preferably assigned a specificity of 75%, which means that 75 out of 100 subjects who do not have caries are correctly assigned as being healthy according to said method. More preferably, the method of the invention has a specificity of 90%, even more preferably 95%, furthermore preferably 99%, yet more preferably 99.9%. Thus, comparing the response variable with a corresponding reference response variable equivalent to a given specificity in a population of subjects without caries, means evaluating whether there is a difference in response variables between that of said subject and that of said population of healthy subjects.

Alternatively, the corresponding reference response variable of a population of subjects with caries is based on *(i.e.* corresponds with) the sensitivity of the method of the invention. As mentioned above, sensitivity is the ability of the method to correctly identify those subjects who have caries from a population of subjects with caries. For example, in the method of the invention the corresponding reference response variable in a population of subjects with caries is preferably assigned a sensitivity of 75%, which means that 75 out of 100 patients who are at risk of caries are correctly assigned as being susceptible to this condition according to said method. More preferably, the method of the invention has a sensitivity of 90%, even more preferably 95%, furthermore preferably 99%, yet more preferably 99.9%. Thus, comparing the response variable with a corresponding reference response variable equivalent to a given sensitivity in a population of subjects with caries, means evaluating whether there is a difference in response variables between that of said subject and that of said population of subjects with caries.

Thus, overall, all selected compounds are measured in a multi-variate analysis to provide a caries risk value of between 0 and 1, wherein 0 represents no risk of caries formation and 1 represents guaranteed caries formation. A subject with a caries risk value between 0.5 and 1 indicates that said subject is at risk of contracting or having caries. This overall value informs the clinician of the general tendency of the individual subject (or patient) to develop caries and therefore the urgency at which treatment, if any, must be provided. Moreover, the combination of measurements is more informative and sensitive than individual ones. For instance, an individual (or patient) may present normal concentration values for a given compound but have out-of-range concentration values for another (see **Tables 2A, 2B, 3, 4, 5A, 5B, 6** and **7).** This is one of the reasons why other tests based on individual variables (see **Figures 9A** to **9C)** are less sensitive to detect caries risk.

In addition, not only the interaction among concentrations of each compound, but the concentration of each compound also may provide information about the caries risk that any given individual (or patient) faces. In this regard, the univariate analysis determines the concentration of salivary compounds from an individual (or patient) that, once measured and compared to the concentrations of salivary compounds from a healthy population or a population with caries, determine the appropriate treatment necessary to prevent caries from forming or to prevent growth of already-existing caries. Thus, a preferred embodiment of the present invention further comprises prescribing a plan of care to a subject determined to have a high caries risk in step (c), wherein said plan of care comprises

(d) comparing at least one concentration used to calculate the response variable compared in step (b) with a corresponding reference concentration in a population of subjects without caries, or comparing at least one concentration used to calculate the response variable compared in step (b) with a corresponding reference concentration in a population of subjects with caries; and

(e) determining the imbalance in said concentration based on the degree of difference in concentration compared in step (d), wherein

(vi) a greater degree of difference between the concentration of the subject and the corresponding reference concentration in a population of subjects without caries, or

(vii) a smaller degree of difference between the concentration of the subject and the corresponding reference concentration in a population of subjects with caries,
is indicative of higher imbalance in said concentration; and

(f) modulating

(viii) the immune system, when a compound whose concentration is determined to have a high imbalance in step (e) is a compound selected from salivary components of the immune system;
(ix) microbial adhesion to a surface of the buccal cavity, when a compound whose concentration is determined to have a high imbalance in step (e) is a compound selected from salivary components involved in microbial adhesion to a surface of the buccal cavity; and/or
(x) metabolism in the buccal cavity or the pH of the buccal cavity, when a compound whose concentration is determined to have a high imbalance in step (e) is a compound selected from salivary components involved in metabolism in the buccal cavity or in regulating the pH of the buccal cavity.

In this aspect of the invention, a subject determined to have a high caries risk may be a subject who has been assigned this status using the method of the present invention or using other methods including clinical assessment, more preferably the method of the present invention. Since the plan of care is determined based on which category each compound, from whose concentration the response variable giving rise to the assignment of said subject as being a high caries risk is calculated, falls into, be it a compound selected from salivary components of the immune system, a compound selected from salivary components involved in microbial adhesion to a surface of the buccal cavity, or a compound selected from salivary components involved in metabolism in the buccal cavity or in regulating the pH of the buccal cavity, the plan of care is a form of targeted therapy which is a subject-specific preventive treatment or therapy which seeks to lower caries risk. These preventive treatments or therapies compensate for the anomalous salivary composition, restoring the features of the healthy oral cavity such as the immune-protective, microbial adhesion or buffering features of saliva. For example, phosphate is found to be one of the concentrations used to determine the high caries risk, then it is possible to modulate metabolism in the buccal cavity or the pH of the buccal cavity, or, more specifically to modulate the phosphate level.

In this embodiment of the present invention, steps (d) and (e) are performed using the concentrations of individual compounds by univariate analysis. Univariate analysis involves comparing at least one concentration determined in step (a), preferably at least one concentration used to calculate the response variable compared in step (b), with the corresponding at least one reference concentration in a population of subjects without caries or the corresponding at least one reference concentration in a population of subjects with caries, and using the difference in said at least one concentration to determine the imbalance in concentration, if any, of said compound in said subject.

Comparing the at least one concentration used to calculate the response variable compared in step (b) with a corresponding reference concentration in a population of subjects without caries, means evaluating whether there is a difference in concentrations between that of said subject and that of said population of healthy subjects. Equally, comparing the at least one concentration used to calculate the response variable compared in step (b) with a corresponding reference concentration in a population of subjects with caries, means evaluating whether there is a difference in concentrations between that of said subject and that of said population of subjects having caries. Said difference is a numerical difference based on a threshold value, preferably a statistically significant difference. When said difference is such that said at least one concentration determined in step (a) is statistically significantly greater than or statistically significantly less than said corresponding reference concentration in a population of subjects without caries, or is statistically significantly greater than or statistically significantly less than said corresponding reference concentration in a population of subjects with caries, this is indicative of an imbalance in concentration of said compound in the subject, *i.e.* that the subject may suffer from an imbalance in the compound.

Alternatively, the corresponding reference concentration in a population of subjects without caries is based on *(i.e.* corresponds with) the specificity of the method of the invention. Specificity is the ability of the method to correctly identify those subjects who do not have caries from a population of healthy subjects *(i.e.* a population of subjects without caries). For example, in the method of the invention the corresponding reference concentration in a population of subjects without caries is preferably assigned a specificity of 75%, which means that 75 out of 100 subjects who do not have caries are correctly assigned as being healthy according to said method. The higher the specificity of the method, the lower chance there will be that a healthy individual is incorrectly classified as caries prone *(i.e.* the caries risk method will be more specific and will produce less false positives). More preferably, the method of the invention has a specificity of 90%, even more preferably 95%, furthermore preferably 99%, yet more preferably 99.9%. Thus, comparing the concentration with a corresponding reference concentration which is equivalent to a given specificity in a population of subjects without caries, means evaluating whether there is a difference in concentrations between that of said subject and that of said population of healthy subjects in order to determine whether or not said subject has an imbalance in concentration of said compound.

Alternatively, the corresponding reference concentration in a population of subjects with caries is based on *(i.e.* corresponds with)* the sensitivity of the method of the invention. Sensitivity is the ability of the method to correctly identify those subjects who have caries from a population of subjects with caries. For example, the method of the invention is preferably assigned a sensitivity of 75%, which means that 75 out of 100 patients who have caries are correctly diagnosed with this condition according to said method. The higher the sensitivity of the method, the lower chance there will be that a diseased individual is not detected *(i.e.* the method will be more sensitive and will produce less false negatives)*. More preferably, the method of the invention has a sensitivity of 90%, even more preferably 95%, furthermore preferably 99%, yet more preferably 99.9%. Thus, comparing the concentration with a corresponding reference concentration which is equivalent to a given sensitivity in a population of subjects without caries, means evaluating whether there is a difference in concentrations between that of said subject and that of said population of subjects with caries in order to determine whether or not said subject has an imbalance in concentration of said compound.

In addition, the aforementioned plan of care comprises modulating (viii) the immune system, when a compound whose concentration is determined to have a high imbalance in step (e) is a compound selected from salivary components of the immune system;

(ix) microbial adhesion to a surface of the buccal cavity, when a compound whose concentration is determined to have a high imbalance in step (e) is a compound selected from salivary components involved in microbial adhesion to a surface of the buccal cavity; and/or

(x) metabolism in the buccal cavity or the pH of the buccal cavity, when a compound whose concentration is determined to have a high imbalance in step (e) is a compound selected from salivary components involved in metabolism in the buccal cavity or in regulating the pH of the buccal cavity. Thus, modulation of the immune system, microbial adhesion to a surface of the buccal cavity, and/or metabolism in the buccal cavity or the pH of the buccal cavity is carried out in a patient having high caries risk depending not only on whether any given compound used to calculate the response variable compared in step (b) is a salivary component of the immune system, is a salivary component involved in microbial adhesion to a surface of the buccal cavity or is a salivary component involved in metabolism in the buccal cavity or the pH of the buccal cavity, respectively, but also on the degree of difference in concentration that said compound has from a corresponding reference concentration in a population of subjects without caries, or from a corresponding reference concentration in a population of subjects with caries.

Thus, the SIMMA test provides not only a general caries risk assessment, but also identifies the likely biological origin of said risk, namely whether it derives from an immune imbalance, and/or tendency to adhesion of cariogenic organisms, and/or lack of acid buffering. Based on the SIMMA test outcome, a preventive, personalized treatment is possible, preferably directed towards one or more of the following goals:

(1) modulation of the immune system to select a non-cariogenic oral biofilm;
(2) modulation of the microbial adhesion to a surface of the buccal cavity by diminishing the adhesion capacity of a cariogenic biofilm;
(3) modulation of the metabolism in the buccal cavity or the pH of the buccal cavity by improving buffering capacity.

The present invention additionally relates to a kit for predicting caries risk in a subject. Said kit is characterized in that it comprises:

(a) means for determining the concentration in saliva from said subject of

i) at least one compound selected from salivary components of the immune system; and
ii) at least one compound selected from salivary components involved in microbial adhesion to a surface of the buccal cavity; and
iii) at least one compound selected from salivary components involved in metabolism in the buccal cavity or in regulating the pH of the buccal cavity;

(b) instructions for comparing a response variable with a corresponding reference response variable in a population of subjects without caries or comparing a response variable with a corresponding reference response variable in a population of subjects with caries, wherein a response variable is calculated from more than one concentration determined in step (a); and

(c) instructions for determining the caries risk based on the degree of difference in response variable compared in step (b), wherein

iv) a greater degree of difference between the response variable of the subject and the corresponding reference response variable in a population of subjects without caries, or

v) a smaller degree of difference between the response variable of the subject and the corresponding reference response variable in a population of subjects with caries,

is indicative of higher caries risk in said subject.

In the kit of the present invention, said means for determining the concentration in saliva from said subject of i) at least one compound selected from salivary components of the immune system; ii) at least one compound selected from salivary components involved in microbial adhesion to a surface of the buccal cavity; and iii) at least one compound selected from salivary components involved in metabolism in the buccal cavity or in regulating the pH of the buccal cavity comprise the aforementioned standard analytical chemical techniques or instruments. The means employed in the kit of the present invention preferably are means used for determining the concentration in saliva of i) at least one compound selected from salivary components of the immune system; ii) at least one compound selected from salivary components involved in microbial adhesion to a surface of the buccal cavity; and iii) at least one compound selected from salivary components involved in metabolism in the buccal cavity or in regulating the pH of the buccal cavity, according to any of the preferred embodiments of the method of the invention described herein.

Moreover, in the kit of the present invention, said instructions for (b) comparing a response variable with a corresponding reference response variable in a population of subjects without caries or comparing a response variable with a corresponding reference response variable in a population of subjects with caries, wherein a response variable is calculated from more than one concentration determined in step (a); and said instructions for (c) determining the caries risk based on the degree of difference in response variable compared in step (b), each comprise mathematical instructions or computer instructions, more preferably at least one algorithm. Preferably, the instructions for comparing according to (b) and the instructions for determining the caries risk according to (c), may be comprised and carried out by the same computer program. Alternatively, the instructions for comparing according to (b) and the instructions for determining the caries risk according to (c), may be carried out by different computer programs.

Analogous with the method of the present invention, in a preferred embodiment the kit of the present invention further comprises instructions for prescribing a plan of care to a subject determined to have a high caries risk in step (c), wherein said plan of care comprises

(d) comparing at least one concentration used to calculate the response variable compared in step (b) with a corresponding reference concentration in a population of subjects without caries, or comparing at least one concentration used to calculate the response variable compared in step (b) with a corresponding reference concentration in a population of subjects with caries; and

(e) determining the imbalance in said concentration based on the degree of difference in concentration compared in step (d), wherein

(vi) a greater degree of difference between the concentration of the subject and the corresponding reference concentration in a population of subjects without caries, or

(vii) a smaller degree of difference between the concentration of the subject and the corresponding reference concentration in a population of subjects with caries,

is indicative of higher imbalance in said concentration; and

(f) modulating

(viii) the immune system, when a compound whose concentration is determined to have a high imbalance in step (e) is a compound selected from salivary components of the immune system;

(ix) microbial adhesion to a surface of the buccal cavity, when a compound whose concentration is determined to have a high imbalance in step (e) is a compound selected from salivary components involved in microbial adhesion to a surface of the buccal cavity; and/or

(x) metabolism in the buccal cavity or the pH of the buccal cavity, when a compound whose concentration is determined to have a high imbalance in step (e) is a compound selected from salivary components involved in metabolism in the buccal cavity or in regulating the pH of the buccal cavity.

The present additionally relates to the use of the method of the present invention or the kit of the present invention, as described herein, in predicting caries risk in a subject.

In conclusion, the SIMMA test of the present invention provides:

1. An overall caries risk value.

2. A list of salivary components that show skewed values.

3. A prescribed plan of care to reduce caries risk (proposed prevention treatment to restore values to the healthy range).

A flow chart of the SIMMAtest, its rationale and applications is shown in **Figure 11.**

**Examples**

I) Material and Methods

a) *Donors selection and sampling procedure*

An intraoral examination was performed on 20 selected subjects, gathering information on: presence of caries, plaque deposits (Oral Hygiene Index, OHI) (Green and Vermillion, 1960), presence of gingival bleeding (Löe and Silness gingival index, GI) (Löe and Silness, 1963), following recommendations and nomenclature from the World Health Organization (WHO, 1997). They had not been treated with antibiotics in the three months prior to the study nor presented antecedents of routine use of oral antiseptics. They were all adults, aged 19 - 39 years, and had no missing teeth. Ten subjects had no history of dental caries (DMF index = 0) and the other ten had active cavities at the moment of sampling, with or without a history of dental caries (fillings). All donors brushed their teeth at 9 - 10 am using water, to prevent any potential effect of toothpaste on salivary composition and characteristics. Non-stimulated saliva samples were taken by drooling at 30 minutes, 6 hours, 12 hours and 24 hours after brushing teeth, collecting it in a sterile 50 mL Falcon tube avoiding spitting or plaque removal by the tongue (Navazesh and Christensen, 1982), up to a total of 5 mL. Samples were immediately frozen at -80 °C until used.

b) *Quantification of salivary immune components*

Based on theoretical or experimental literature precedent suggesting that they could each, individually, be related to caries initiation, 24 components present in saliva were selected for quantitative measurement in saliva samples from caries-bearing (CA) and caries-free (NOCA) individuals (Lenander-Lumikari and Loimaranta 2000, Leone and Oppenheim 2001, Preethi et al. 2010, Kawas et al. 2011, Guo and Shi 2013, Cheaib and Lussi 2013). Accordingly, enzyme-linked immunosorbent assays (ELISA) and colorimetric tests were performed in duplicate to measure the salivary concentrations of 24 components, following the manufacturer's recommendations. A list of all 24 components, including the manufacturer information and the sample dilution used is shown in **Table 1.**

**Table 1.** Salivary components measured

|  | Salivary Component | Manufacturer | Sample dilution |
|---|---|---|---|
|  | IgA | Assaypro | 1:1000 |
|  | IgG | Assaypro | 1:10 |
|  | IgM | Assaypro | 1 |
|  | Alpha-defensin 1-3 | Assaypro | 1:10 |
|  | Beta-defensin 1 | Sun Red | 1 |
|  | Beta-defensin 2 | Sun Red | 1 |
| Salivary Immune System Components | Beta-defensin 3 | Sun Red | 1 |
|  | Cathelicidin LL-37 | Assaypro | 1:200 |
|  | Lactoferrin | Assaypro | 1:20 |
|  | Calprotectin | Assaypro | 1 |
|  | C3a | Sun Red | 1:10 |
|  | Lysozyme | Assaypro | 1:8000 |

(continued)

| | Salivary Component | Manufacturer | Sample dilution |
|---|---|---|---|
| Salivary Adhesion Components | PRB1 | Sun Red | 1:10 |
| | Statherin | Sun Red | 1:200 |
| | Collagen type I | Sun Red | 1 |
| | Mucin 7 | Sun Red | 1:50 |
| | Mucin 5B | Sun Red | 1:100 |
| | Alpha-2 macroglobulin | Assaypro | 1:4 |
| | Fibronectin | Assaypro | 1:20 |
| Salivary Acidity-regulating Components | Lactate | BioVision | 1:20 |
| | Formate | BioVision | 1:5 |
| | Calcium | BioVision | 1 |
| | Phosphate | BioVision | 1:200 |
| | Urea | BioVision | 1:25 |

c) *Statistical analysis*

Two requirements in terms of usability expectations and model robustness were focused on: First, to include in the model at least two variables of each of the groups salivary immune system components, salivary adhesion components and salivary acidity-regulating components. Second, to be able to provide to the patient two different and complementary risk measurements: on the one hand a univariant-obtained local risk based on the comparison of the patient's values of selected variables to confidence intervals calculated on caries-free individuals, and on the other hand a multivariant-obtained global risk provided by the overall model. Local-wise analysis allows design of modular therapies focused on one of the salivary adhesion, acidity-regulating and immune-system component groups, while global-wise analysis provides an emergency degree for treatment, in order to take those variables out of confidence intervals back to normal values.

In order to select the variables included in the model disclosed herein, the Wilcoxon test, as implemented in R environment (R Core Team 2014), was performed on each of the candidate variables. The lower the p-value provided by the test the higher is the capability of a variable to distinguish between two groups of samples [Caries (CA) and No-Caries (NOCA)]. A non-parametric approach was adopted to avoid making assumptions about variables distribution. Apart from exhibiting a significant p-value, a requirement of a variable to be included in the model disclosed herein is that confidence intervals (given by lower and upper quartiles) corresponding to groups of samples Caries and No-Caries do not overlap.

In order to assess the classification accuracy of the variables selected, the k-fold cross-validation technique as implemented in 'galgo' R package (Trevino & Falciani 2014) was adopted. The dataset was split into k different training and test sets and the classification accuracy was then defined as the average of the classification accuracies of a model trained on training sets and calculated on the test sets for each of the k splits. Currently, only the set of variables to be included in the model is known but nothing is known about their interactions structure. For this reason a single-hidden-layer neural network modelling implemented in 'nnet' R package (Venables & Ripley 2002) and offered by 'galgo' as unsupervised approach to calculate classification accuracy of the variables selected was adopted.

In addition, the structural inference of this model was addressed following a genetic algorithm screening as implemented in 'glmulti' R package (Calgano 2013) that allows only appropriate and essential interactions between selected variables to be included in the model. Once the structure of our model was defined, global risks were predicted from new observations following a supervised approach. The reason for adopting a supervised approach (instead of the unsupervised approach used above) is that the response variable to be explained is a binary variable (Caries or No-Caries) which naturally follows a binomial distribution. A supervised approach allows such an assumption to be made and in this way it is easy to provide a global risk or probability given an observation by parameterizing the model in terms of the parameter p of the binomial distribution.

**Example 1:** *Selection of sampling time*

An initial test was performed with 10 salivary immune system proteins (IgA, IgG, IgM, Alpha-defensin 1-3, Beta-defensin 1, Beta-defensin 2, Beta-defensin 3, LL-37, Lactoferrin and Calprotectin), the concentrations of which were measured using ELISA in saliva samples taken at 0.5, 6, 12 and 24 hours after brushing teeth in order to establish the optimal sampling time. Significant variability was observed over time, indicating that the salivary concentration of these proteins is not constant. Thus, immune molecules that could be good biomarkers of the disease at a given time may not discriminate between healthy and caries-risk individuals at another time point. It is hypothesized that this may be one of the reasons why the results of commercial salivary tests, that do not specify the sampling time, may not be accurate or consistent.

Given that 12 and 24 hours after brushing teeth will not represent a comfortable and reliable sampling time for clinical use, and that differences between caries-bearing and caries-free individuals were lowest at these sampling times, the measurements of all 24 salivary components were performed at 0.5 and 6 hours after brushing teeth. The measured concentrations of the 24 selected salivary components from the three categories for caries-free and caries-bearing individuals are indicated in **Tables 2A and 2B, 3** and **4** (values at 0.5 hours after brushing teeth), and **Tables 5A and 5B, 6,** and **7** (values at 6 hours after brushing teeth).

**Table 2A:** Concentrations of salivary immune system components at 0.5 hours

|  | C3a (µg/mL) | Alpha-Defensin 1-3 (ng/mL) | Beta-Defensin 1 (ng/mL) | Beta-Defensin 2 (pg/mL) | Beta-Defensin 3 (ng/mL) |
|---|---|---|---|---|---|
| **CA 01** | 17.14 | 1673.48 | 14.44 | 281.53 | 15.53 |
| **CA 02** | 19.90 | 1442.43 | 21.23 | 434.07 | 5.74 |
| **CA 03** | 14,05 | 1331.31 | 6.77 | 258.57 | 11.55 |
| **CA 04** | 6.70 | 1324.82 | 12.55 | 246.73 | 12.70 |
| **CA 05** | 14.94 | 1359.21 | 13.81 | 215.88 | 14.97 |
| **CA 06** | 18.52 | 922.43 | 9.32 | 238.75 | 12.30 |
| **CA 07** | 16.11 | 1158.55 | 14.01 | 241.99 | 6.30 |
| **CA 08** | 13.23 | 863.30 | 11.42 | 243.40 | 3.10 |
| **CA 09** | 9.37 | 1000.68 | 8.96 | 218.73 | 17.30 |
| **CA 10** | 21.00 | 800.12 | 3.56 | 241.97 | 3.50 |
| **NOCA 01** | 18.39 | 946.83 | 13.84 | 237.93 | 6.30 |
| **NOCA 02** | 33.68 | 180.48 | 5.32 | 142.80 | 3.10 |
| **NOCA 04** | 20.51 | 1115.92 | 12.92 | 243.30 | 17.20 |
| **NOCA 05** | 14.65 | 721.72 | 9.36 | 243.64 | 10.90 |
| **NOCA 07** | 20.82 | 909.21 | 6.16 | 265.87 | 5.74 |
| **NOCA 08** | 9.27 | 979.52 | 14.13 | 216.34 | 11.55 |
| **NOCA 09** | 8.16 | 1236.32 | 14.31 | 250.22 | 15.86 |
| **NOCA 10** | 15.22 | 964.35 | 4.17 | 232.12 | 8.80 |
| **NOCA 11** | 16.15 | 1058.83 | 6.67 | 374.13 | 2.46 |
| **NOCA 13** | 21.57 | 1030.00 | 6.73 | 294.02 | 7.56 |

CA = caries patient (e.g. CA 99 represents caries patient 99)
NOCA = non-caries (healthy) patient

**Table 2B:** Concentrations of salivary immune system components at 0.5 hours

|  | Lactof. (mg/L) | LL-37 (ng/mL) | Calprot. (µg/mL) | IgA (mg/dL) | IgG (mg/dL) | IgM (mg/dL) | Lysozyme (ng/mL) |
|---|---|---|---|---|---|---|---|
| **CA 01** | 3.88 | 101.19 | 2.47 | 41.59 | 6.24 | 0.07 | 3274.38 |
| **CA 02** | 5.45 | 109.11 | 2.05 | 18.41 | 5.59 | 0.01 | 7367.50 |
| **CA 03** | 5.83 | 126.87 | 3.03 | 56.09 | 6.24 | 0.07 | 0.00 |
| **CA 04** | 2.41 | 117.35 | 1.83 | 62.24 | 5.59 | 0.01 | 4790.92 |
| **CA 05** | 11.59 | 175.29 | 2.87 | 38.92 | 6.12 | 0.08 | 603.16 |
| **CA 06** | 9.98 | 129.30 | 3.13 | 41.41 | 8.05 | 0.21 | 0.00 |
| **CA 07** | 13.01 | 181.12 | 0.22 | 63.98 | 7.00 | 0.22 | 0.00 |
| **CA 08** | 9.23 | 145.61 | 3.13 | 21.96 | 7.46 | 0.21 | 0.00 |
| **CA 09** | 10.97 | 144.09 | 1.20 | 10.47 | 8.78 | 0.18 | 0.00 |

(continued)

|  | Lactof. (mg/L) | LL-37 (ng/mL) | Calprot. ($\mu$g/mL) | IgA (mg/dL) | IgG (mg/dL) | IgM (mg/dL) | Lysozyme (ng/mL) |
|---|---|---|---|---|---|---|---|
| CA 10 | 8.45 | 219.16 | 0.72 | 12.58 | 8.24 | 0.17 | 2129.46 |
| NOCA 01 | 10.28 | 113.90 | 0.74 | 59.71 | 8.06 | 0.19 | 949.11 |
| NOCA 02 | 1.49 | 117.67 | 0.31 | 25.36 | 0.73 | 0.04 | 6695.33 |
| NOCA 04 | 12.46 | 116.18 | 2.35 | 64.31 | 8.20 | 0.22 | 288.70 |
| NOCA 05 | 7.49 | 133.83 | 0.43 | 54.57 | 8.23 | 0.21 | 0.00 |
| NOCA 07 | 5.27 | 92.05 | 0.75 | 66.42 | 3.74 | 0.22 | 5189.01 |
| NOCA 08 | 3.40 | 92.72 | 0.65 | 57.67 | 5.38 | 0.22 | 2077.54 |
| NOCA 09 | 6.90 | 101.87 | 3.59 | 65.60 | 5.58 | 0.01 | 1554.40 |
| NOCA 10 | 10.51 | 113.34 | 0.29 | 64.11 | 7.97 | 0.16 | 0.00 |
| NOCA 11 | 4.16 | 94.06 | 4.98 | 58.74 | 6.28 | 0.21 | 1244.03 |
| NOCA 13 | 4.85 | 89.28 | 2.47 | 64.71 | 6.62 | 0.01 | 0.00 |

Lactof. = Lactoferrin LL-37 = Cathelicidin LL-37 Calprot. = Calprotectin CA = caries patient (e.g. CA 99 represents caries patient 99) NOCA = non-caries (healthy) patient

**Table 3:** Concentrations of salivary adhesion proteins at 0.5 hours

|  | A2M ($\mu$g/mL) | MUC5B (ng/mL) | MUC7 (ng/mL) | PRB1 (ng/mL) | Statherin (ng/mL) | Collagen I ($\mu$g/mL) | Fibr. ($\mu$g/mL) |
|---|---|---|---|---|---|---|---|
| CA 01 | $1.5 \times 10^{-11}$ | 1989.44 | 2407.14 | 81.62 | 18898.71 | 45.60 | 0.13 |
| CA 02 | $9.8 \times 10^{-3}$ | 1839.20 | 2366.23 | 95.75 | 17076.29 | 36.31 | 5.51 |
| CA 03 | $3.3 \times 10^{-4}$ | 1919.02 | 2030.89 | 75.02 | 17973.00 | 18.37 | 4.35 |
| CA 04 | $2.2 \times 10^{-2}$ | 1856.06 | 1941.80 | 87.29 | 16271.43 | 9.00 | 2.81 |
| CA 05 | $2.4 \times 10^{-3}$ | 2148.50 | 2634.64 | 83.06 | 20224.43 | 19.45 | 4.91 |
| CA 06 | $2.9 \times 10^{-2}$ | 2140.77 | 2509.98 | 103.83 | 19164.43 | 28.83 | 9.37 |
| CA 07 | $3.1 \times 10^{-2}$ | 2037.44 | 2186.68 | 98.18 | 18055.29 | 38.46 | 1.95 |
| CA 08 | $2.9 \times 10^{-3}$ | 1877.02 | 2304.86 | 80.08 | 19424.43 | 19.43 | 0.98 |
| CA 09 | $4.6 \times 10^{-3}$ | 2212.14 | 2757.82 | 71.75 | 24071.57 | 18.60 | 1.58 |
| CA 10 | $1.9 \times 10^{-6}$ | 1993.80 | 2478.73 | 68.61 | 19951.57 | 35.16 | 1.27 |
| NOCA 01 | $5.6 \times 10^{-10}$ | 1603.71 | 2004.64 | 59.09 | 19174.43 | 30.50 | 0.21 |
| NOCA 02 | 0.0 | 1763.74 | 1766.95 | 69.52 | 19403.00 | 71.21 | 0.02 |
| NOCA 04 | $2.1 \times 10^{-10}$ | 2047.29 | 2461.91 | 75.17 | 22210.14 | 38.35 | 0.50 |
| NOCA 05 | $7.6 \times 10^{-7}$ | 2027.14 | 2497.59 | 91.18 | 22523.00 | 27.74 | 0.64 |
| NOCA 07 | $3.2 \times 10^{-2}$ | 2086.68 | 2142.93 | 103.78 | 23264.43 | 35.03 | 3.78 |
| NOCA 08 | $2.7 \times 10^{-2}$ | 1905.77 | 2183.05 | 85.91 | 20101.57 | 22.07 | 6.57 |
| NOCA 09 | $1.8 \times 10^{-2}$ | 1549.35 | 2399.52 | 94.41 | 20928.71 | 19.79 | 2.25 |
| NOCA 10 | $6.0 \times 10^{-11}$ | 1685.05 | 2232.02 | 76.32 | 18641.57 | 32.12 | 0.29 |
| NOCA 11 | $2.5 \times 10^{-2}$ | 2017.56 | 2176.68 | 91.93 | 19971.57 | 35.03 | 1.65 |
| NOCA 13 | $2.6 \times 10^{-9}$ | 1976.38 | 2429.41 | 100.38 | 19641.57 | 38.42 | 0.22 |

A2M = Alpha 2 Macroglobulin MUC5B = Mucin 5B MUC7 = Mucin 7 Fibr. = Fibronectin CA = caries patient (e.g. CA 99 represents caries patient 99) NOCA = non-caries (healthy) patient

**Table 4:** Concentrations of salivary acidity-regulating components at 0.5 hours

|  | Calcium (mg/mL) | Formate ($\mu$mol/mL) | Phosphate ($\mu$mol/mL) | Lactate ($\mu$mol/mL) | Urea ($\mu$mol/mL) |
|---|---|---|---|---|---|
| **CA 01** | 0.036 | 0.00 | 53.33 | 0.29 | 2.07 |
| **CA 02** | 0.021 | 0.25 | 49.63 | 2.54 | 2.29 |
| **CA 03** | 0.017 | 0.00 | 46.61 | 0.30 | 2.41 |
| **CA 04** | 0.009 | 0.01 | 56.80 | 0.00 | 2.26 |
| **CA 05** | 0.014 | 0.00 | 59.48 | 1.84 | 2.14 |
| **CA 06** | 0.025 | 0.07 | 50.94 | 0.14 | 2.32 |
| **CA 07** | 0.035 | 0.10 | 41.28 | 0.00 | 2.28 |
| **CA 08** | 0.036 | 0.00 | 48.04 | 4.08 | 2.40 |
| **CA 09** | 0.016 | 0.00 | 59.38 | 3.55 | 1.70 |
| **CA 10** | 0.021 | 0.00 | 50.82 | 2.99 | 2.18 |
| **NOCA 01** | 0.024 | 0.03 | 51.02 | 0.99 | 2.35 |
| **NOCA 02** | 0.029 | 0.00 | 52.90 | 0.37 | 2.40 |
| **NOCA 04** | 0.029 | 0.17 | 59.38 | 3.14 | 2.11 |
| **NOCA 05** | 0.011 | 0.51 | 50.82 | 1.78 | 2.43 |
| **NOCA 07** | 0.027 | 0.00 | 51.02 | 0.00 | 2.42 |
| **NOCA 08** | 0.037 | 0.00 | 52.90 | 0.00 | 2.16 |
| **NOCA 09** | 0.024 | 0.00 | 63.41 | 0.00 | 2.05 |
| **NOCA 10** | 0.013 | 0.70 | 57.60 | 0.39 | 2.20 |
| **NOCA 11** | 0.022 | 0.02 | 58.78 | 0.00 | 2.34 |
| **NOCA 13** | 0.025 | 0.00 | 55.37 | 1.39 | 2.38 |

CA = caries patient (e.g. CA 99 represents caries patient 99)
NOCA = non-caries (healthy) patient

**Table 5A:** Concentrations of salivary immune system components at 6 hours

|  | C3a ($\mu$g/mL) | Alpha-Defensin 1-3 (ng/mL) | Beta-Defensin 1 (ng/mL) | Beta-Defensin 2 (pg/mL) | Beta-Defensin 3 (ng/mL) |
|---|---|---|---|---|---|
| **CA 01** | 18.29 | 1212.95 | 8.34 | 248.27 | 3.56 |
| **CA 02** | 22.00 | 1434.36 | 14.21 | 381.24 | 2.79 |
| **CA 03** | 15.40 | 1363.43 | 14.20 | 399.94 | 6.21 |
| **CA 04** | 24.09 | 1418.16 | 6.55 | 341.88 | 7.97 |
| **CA 05** | 14.44 | 1448.06 | 21.90 | 333.58 | 4.92 |
| **CA 06** | 13.28 | 1006.01 | 14.76 | 227.27 | 10.40 |
| **CA 07** | 9.42 | 1020.13 | 13.27 | 244.97 | 3.00 |
| **CA 08** | 18.80 | 986.07 | 18.20 | 247.06 | 3.00 |
| **CA 09** | 17.45 | 950.31 | 8.23 | 242.76 | 5.70 |
| **CA 10** | 21.14 | 926.54 | 15.78 | 235.79 | 3.80 |
| **NOCA 01** | 22.38 | 992.63 | 15.79 | 44.52 | 3.00 |
| **NOCA 02** | 27.60 | 104.29 | 8.87 | 32.43 | 3.00 |
| **NOCA 04** | 15.73 | 1039.86 | 17.99 | 243.46 | 17.10 |
| **NOCA 05** | 16.84 | 969.34 | 3.59 | 115.83 | 4.50 |
| **NOCA 07** | 20.14 | 1231.68 | 10.16 | 187.08 | 2.79 |
| **NOCA 08** | 19.26 | 1764.42 | 18.00 | 59.83 | 6.21 |
| **NOCA 09** | 17.62 | 1407.80 | 11.16 | 119.35 | 17.97 |
| **NOCA 10** | 18.81 | 1030.50 | 14.83 | 109.20 | 4.70 |
| **NOCA 11** | 15.11 | 1665.22 | 4.51 | 162.26 | 2.65 |

(continued)

|  | C3a (μg/mL) | Alpha-Defensin 1-3 (ng/mL) | Beta-Defensin 1 (ng/mL) | Beta-Defensin 2 (pg/mL) | Beta-Defensin 3 (ng/mL) |
|---|---|---|---|---|---|
| **NOCA 13** | 21.33 | 1128.57 | 14.71 | 136.30 | 5.71 |

CA = caries patient (e.g. CA 99 represents caries patient 99)
NOCA = non-caries (healthy) patient

**Table 5B:** Concentrations of salivary immune system components at 6 hours

|  | Lactof. (mg/L) | LL-37 (ng/mL) | Calprot. (μg/mL) | IgA (mg/dL) | IgG (mg/dL) | IgM (mg/dL) | Lysozyme (ng/mL) |
|---|---|---|---|---|---|---|---|
| **CA 01** | 4.94 | 149.77 | 2.46 | 28.81 | 6.08 | 0.14 | 13381.80 |
| **CA 02** | 6.51 | 99.30 | 2.17 | 41.10 | 5.56 | 0.18 | 2969.12 |
| **CA 03** | 2.87 | 162.91 | 2.98 | 35.50 | 6.08 | 0.16 | 398.61 |
| **CA 04** | 6.54 | 116.96 | 3.04 | 52.95 | 5.56 | 0.11 | 6823.59 |
| **CA 05** | 8.10 | 130.48 | 2.72 | 17.06 | 6.52 | 0.04 | 766.33 |
| **CA 06** | 11.04 | 137.80 | 3.12 | 62.40 | 8.57 | 0.23 | 286.49 |
| **CA 07** | 11.22 | 132.84 | 0.16 | 53.08 | 8.49 | 0.21 | 28283.58 |
| **CA 08** | 10.78 | 188.79 | 3.93 | 42.02 | 7.34 | 0.22 | 5258.16 |
| **CA 09** | 10.33 | 191.68 | 1.99 | 8.59 | 8.76 | 0.18 | 1081.47 |
| **CA 10** | 10.03 | 140.65 | 1.77 | 7.88 | 8.04 | 0.18 | 2404.57 |
| **NOCA 01** | 10.87 | 145.94 | 0.84 | 47.08 | 2.31 | 0.14 | 17815.12 |
| **NOCA 02** | 0.79 | 81.09 | 0.64 | 17.95 | 0.18 | 0.04 | 9814.05 |
| **NOCA 04** | 11.47 | 87.33 | 2.73 | 52.83 | 8.82 | 0.22 | 119.52 |
| **NOCA 05** | 10.57 | 96.79 | 1.90 | 18.87 | 7.26 | 0.18 | 1066.56 |
| **NOCA 07** | 8.40 | 88.47 | 0.91 | 64.05 | 4.63 | 0.21 | 12603.77 |
| **NOCA 08** | 2.97 | 89.91 | 0.86 | 61.38 | 5.05 | 0.09 | 6457.80 |
| **NOCA 09** | 6.61 | 92.48 | 5.91 | 61.79 | 6.24 | 0.22 | 19236564.36 |
| **NOCA 10** | 11.35 | 134.90 | 0.57 | 19.51 | 8.00 | 0.18 | 142520997.19 |
| **NOCA 11** | 6.54 | 102.99 | 3.31 | 51.44 | 5.45 | 0.21 | 78512404.22 |
| **NOCA 13** | 5.39 | 90.75 | 2.46 | 38.52 | 6.30 | 0.02 | 0 |

Lactof. = Lactoferrin
LL-37 = Cathelicidin LL-37
Calprot. = Calprotectin
CA = caries patient (e.g. CA 99 represents caries patient 99)
NOCA = non-caries (healthy) patient

**Table 6:** Concentrations of salivary adhesion proteins at 6 hours

|  | A2M (μg/mL) | MUC5B (ng/mL) | MUC7 (ng/mL) | PRB1 (ng/mL) | Statherin (ng/mL) | Collagen I (μg/mL) | Fibr. (μg/mL) |
|---|---|---|---|---|---|---|---|
| **CA 01** | $3.6 \times 10^{-17}$ | 1886.38 | 2294.52 | 84.44 | 19797.29 | 46.60 | 0.16 |
| **CA 02** | $7.5 \times 10^3$ | 1945.77 | 2438.61 | 99.38 | 18035.86 | 66.61 | 3.12 |
| **CA 03** | $9.6 \times 10^3$ | 1792.88 | 1934.98 | 90.54 | 19091.57 | 23.80 | 3.12 |
| **CA 04** | $6.3 \times 10^{-5}$ | 1884.85 | 1923.61 | 81.29 | 18532.86 | 35.43 | 0.67 |
| **CA 05** | $1.1 \times 10^{-8}$ | 2206.68 | 2564.64 | 94.56 | 18233.00 | 31.52 | 0.19 |
| **CA 06** | $1.7 \times 10^{-2}$ | 1967.18 | 2384.86 | 95.02 | 18907.29 | 28.52 | 4.57 |
| **CA 07** | $1.4 \times 10^{-2}$ | 1871.50 | 2288.95 | 74.87 | 18735.86 | 19.14 | 1.18 |
| **CA 08** | $4.0 \times 10^{-3}$ | 2181.98 | 2227.02 | 100.31 | 20177.29 | 31.74 | 0.60 |
| **CA 09** | $8.8 \times 10^{-3}$ | 1797.14 | 2680.89 | 104.04 | 21285.86 | 32.40 | 1.16 |

(continued)

| | A2M (µg/mL) | MUC5B (ng/mL) | MUC7 (ng/mL) | PRB1 (ng/mL) | Statherin (ng/mL) | Collagen I (µg/mL) | Fibr. (µg/mL) |
|---|---|---|---|---|---|---|---|
| **CA 10** | $1.7 \times 10^{-5}$ | 2014.71 | 2598.61 | 90.47 | 20501.57 | 38.53 | 0.66 |
| **NOCA 01** | $5.0 \times 10^{-8}$ | 1860.61 | 2452.14 | 91.22 | 20528.71 | 39.23 | 0.11 |
| **NOCA 02** | 0.0 | 2015.59 | 1861.56 | 72.76 | 19593.00 | 63.02 | 0.01 |
| **NOCA 04** | $3.0 \times 10^{-3}$ | 2112.74 | 2397.36 | 92.76 | 20503.00 | 32.35 | 3.17 |
| **NOCA 05** | $1.9 \times 10^{-4}$ | 1982.74 | 2517.14 | 101.19 | 21044.43 | 26.68 | 0.26 |
| **NOCA 07** | $2.9 \times 10^{-2}$ | 2097.89 | 2613.73 | 107.67 | 20463.00 | 250.35 | 0.61 |
| **NOCA 08** | $1.9 \times 10^{-2}$ | 2367.89 | 2452.14 | 108.44 | 20001.57 | 37.32 | 2.75 |
| **NOCA 09** | $5.2 \times 10^{-3}$ | 1735.89 | 2480.20 | 94.49 | 18088.71 | 41.11 | 0.57 |
| **NOCA 10** | $2.8 \times 10^{-3}$ | 1933.35 | 2426.11 | 109.51 | 23120.14 | 43.25 | 0.19 |
| **NOCA 11** | $8.2 \times 10^{-3}$ | 2015.92 | 2109.52 | 108.75 | 19798.71 | 40.78 | 0.19 |
| **NOCA 13** | $2.0 \times 10^{-10}$ | 1771.41 | 2452.48 | 90.52 | 17885.86 | 1929.43 | 0.19 |

A2M = Alpha 2 Microglobulin MUC5B = Mucin 5B MUC7 = Mucin 7 Fibr. = Fibronectin CA = caries patient (e.g. CA 99 represents caries patient 99) NOCA = non-caries (healthy) patient

**Table 7:** Concentrations of salivary acidity-regulating components at 6 hours

| | Calcium (mg/mL) | Formate (µmol/mL) | Phosphate (µmol/mL) | Lactate (µmol/mL) | Urea (µmol/mL) |
|---|---|---|---|---|---|
| **CA 01** | 0.03 | 0.00 | 44.06 | 0.24 | 2.06 |
| **CA 02** | 0.02 | 0.42 | 50.27 | 0.43 | 2.32 |
| **CA 03** | 0.02 | 0.45 | 50.61 | 1.06 | 2.52 |
| **CA 04** | 0.03 | 0.00 | 57.87 | 2.45 | 2.18 |
| **CA 05** | 0.01 | 0.20 | 50.78 | 1.38 | 2.37 |
| **CA 06** | 0.02 | 0.43 | 50.59 | 0.81 | 2.31 |
| **CA 07** | 0.05 | 0.71 | 52.83 | 8.69 | 2.33 |
| **CA 08** | 0.04 | 0.16 | 60.94 | 2.53 | 1.49 |
| **CA 09** | 0.01 | 0.40 | 51.74 | 5.26 | 1.65 |
| **CA 10** | 0.02 | 0.00 | 48.11 | 0.36 | 1.52 |
| **NOCA 01** | 0.02 | 0.00 | 52.70 | 0.41 | 2.33 |
| **NOCA 02** | 0.02 | 0.00 | 61.10 | 0.47 | 1.32 |
| **NOCA 04** | 0.01 | 0.00 | 51.74 | 0.42 | 2.34 |
| **NOCA 05** | 0.02 | 0.53 | 48.11 | 4.87 | 2.37 |
| **NOCA 07** | 0.02 | 0.00 | 52.70 | 0.43 | 2.40 |
| **NOCA 08** | 0.02 | 0.00 | 61.10 | 0.00 | 0.37 |
| **NOCA 09** | 0.04 | 0.00 | 57.70 | 0.00 | 2.30 |
| **NOCA 10** | 0.01 | 0.00 | 58.51 | 0.00 | 2.41 |
| **NOCA 11** | 0.02 | 0.00 | 53.28 | 5.19 | 2.39 |
| **NOCA 13** | 0.02 | 0.00 | 60.57 | 0.70 | 2.21 |

CA = caries patient (e.g. CA 99 represents caries patient 99) NOCA = non-caries (healthy) patient

**Example 2:** *Selection of caries-associated biomarkers*

The means, standard variations and upper/lower quartiles of all salivary components in caries-free and caries-bearing individuals are shown in **Figure 1** (salivary immune system molecules), **Figure 2** (salivary adhesion molecules) and **Figure 3** (salivary acidity-regulating components) for measurements made 0.5 hours after brushing teeth; and **Figure 4** (salivary immune system molecules), **Figure 5** (salivary adhesion molecules) and **Figure 6** (salivary acidity-regulating components) for measurements made 6 hours after brushing teeth.

Wilcox univariate tests were performed to compare the values between individuals with and without caries **(Table 8).** As can be observed, few of the measured variables in fact have diagnostic value, even if they belong to the same category, indicating that it was not possible to predict a *priori* which salivary components were going to show significant differences.

**Table 8:** p-Values from univariate analyses

| | Time after brushing teeth | 0.5 hours | 6 hours |
|---|---|---|---|
| **Salivary Immune System Proteins** | Cathelicidin LL-37 | 0.007 | 0.002 |
| | IgA | 0.007 | 0.353 |
| | Alpha-Defensin 1-3 | 0.105 | 0.853 |
| | IgM | 0.326 | 1.000 |
| | Beta-Defensin 1 | 0.393 | 0.796 |
| | Lactoferrin | 0.393 | 1.000 |
| | C3a | 0.393 | 0.315 |
| | Calprotectin | 0,405 | 0.326 |
| | Beta-Defensin 3 | 0.520 | 0.970 |
| | IgG | 0.520 | 0.121 |
| | Beta-Defensin 2 | 0.971 | $7.57 \times 10^{-5}$ |
| | Lysozyme | 0.796 | 0.280 |
| **Salivary Adhesion Proteins** | Statherin | 0.063 | 0.247 |
| | Fibronectin | 0.123 | 0.121 |
| | Mucin 5B | 0.218 | 0.684 |
| | Mucin 7 | 0.247 | 0.571 |
| | Collagen I | 0.273 | 0.104 |
| | Alpha-2 Macroglobulin | 0.393 | 0.796 |
| | PRB1 | 0.912 | 0.190 |
| **Salivary Acidity-regulating Components** | Phosphate | 0.140 | 0.054 |
| | Lactate | 0.353 | 0.123 |
| | Urea | 0.393 | 0.393 |
| | Calcium | 0.631 | 0.315 |
| | Formate | 0.853 | 0.029 |

The data show that concentrations of salivary immune system components exhibit better discrimination between the healthy and diseased individuals. At 6 hours, several components from each of the three categories were different between the two patient groups, whereas at 0.5 hours, no differences were found in the salivary (immune system) protein component category. The latter is probably due to the fact that these proteins are mainly produced after dietary carbohydrates fermentation, and are more readily measured at 6 hours (after lunch in the sampling schedule).

Thus, based on the p-values from the univariate analyses **(Table 8)** and the lack of overlap between the data dispersion boxes **(Figures 1** through **6)** the following salivary metabolites were selected to provide discrimination value between healthy and caries-prone individuals:

1. At 6 hours after brushing teeth:

a. Immune system molecules: Beta-defensin 2 and LL-37.

b. Adhesion molecules: Collagen I and Fibronectin.

c. Acidity-regulating (metabolic) components: Formate and Phosphate.

2. At 0.5 hours after brushing teeth:

a. Immune system molecules: LL-37 and IgA.

b. Adhesion molecules: Statherin and Fibronectin.

c. Acidity-regulating (metabolic) components: Phosphate and Lactate.

As can be seen, the concentrations of the aforementioned salivary components with the lowest p-values exhibit different data dispersion in each patient group at 0.5 hours **(Table 9** and **Figure 7)** and 6 hours **(Table 10** and **Figure 8)** after brushing teeth.

**Table 9:** Data dispersion of concentrations (nM) of salivary components in populations of patients with and without caries at 0.5 hours

| | Salivary component | Patient type | Lower quartile | Median | Upper quartile |
|---|---|---|---|---|---|
| **Salivary Immune System Proteins** | Cathelicidin LL-37 | Caries | 117 | 137 | 175 |
| | (ng/mL) | No caries | 92.7 | 108 | 116 |
| | IgA | Caries | 18.4 | 40.2 | 56.1 |
| | (mg/dL) | No caries | 57.7 | 61.9 | 64.7 |
| **Salivary Adhesion Proteins** | Fibronectin | Caries | 1.27 | 2.38 | 4.91 |
| | ($\mu$g/mL) | No caries | 0.221 | 0.569 | 2.25 |
| | Statherin | Caries | 17973 | 19032 | 19952 |
| | (ng/mL) | No caries | 19403 | 20037 | 22210 |
| **Salivary Acidity-regulating Components** | Phosphate | Caries | 48 | 50.9 | 56.8 |
| | ($\mu$mol/mL) | No caries | 51 | 54.1 | 58.8 |
| | Lactate | Caries | 0.141 | 1.07 | 2.99 |
| | ($\mu$mol/mL) | No caries | 0 | 0.383 | 1.39 |

**Table 10:** Data dispersion of concentrations (nM) of salivary components in populations of patients with and without caries at 6 hours

| | Salivary component | Patient type | Lower quartile | Median | Upper quartile |
|---|---|---|---|---|---|
| **Salivary Immune System Proteins** | Cathelicidin LL-37 | Caries | 130 | 139 | 163 |
| | (ng/mL) | No caries | 88.5 | 91.6 | 103 |
| | Beta-Defensin 2 | Caries | 243 | 248 | 342 |
| | (pg/mL) | No caries | 59.8 | 118 | 162 |
| **Salivary Adhesion Proteins** | Fibronectin | Caries | 0.603 | 0.915 | 3.12 |
| | ($\mu$g/mL) | No caries | 0.191 | 0.226 | 0.612 |
| | Collagen I | Caries | 28.5 | 31.7 | 38.5 |
| | ($\mu$g/mL) | No caries | 37.3 | 40 | 41.1 |

(continued)

| Salivary component | Patient type | Lower quartile | Median | Upper quartile |
|---|---|---|---|---|
| **Salivary Acidity-regulating Components** | | | | |
| Phosphate (μmol/mL) | Caries | 50.3 | 50.7 | 52.8 |
| | No caries | 52.7 | 55.5 | 60.6 |
| Formate (μmol/mL) | Caries | 0 | 0.3 | 0.429 |
| | No caries | 0 | 0 | 0 |

The classification accuracy of the selected variables was measured by a cross-validation unsupervised approach (Trevino and Falciani, 2014), indicating that, on average, 98% of the caries individuals are detected by the test. Increasing the number of variables from the 6 selected above to the 8 most significantly different compounds did not improve this percentage.

In contrast, when saliva samples were obtained from the aforementioned subjects according to the protocols employed in commercially-available kits for detecting caries risk in patients, which are based on measurement of the concentration of IgA (using ELISA), pH or buffer capacity (using a pH meter), the resulting data poorly differentiates between populations of healthy and caries-prone individuals (Table 11 and **Figure 9).**

**Table 11:** Data dispersion of concentration of IgA, pH and buffer capacity in populations of patients with and without caries

| Variable | Patient type | Minimum | Lower quartile | Median | Upper quartile | Maximum |
|---|---|---|---|---|---|---|
| [IgA] (μg/mL) | Caries | 368.95 | 369.34 | 369.90 | 370.64 | 371.42 |
| | No caries | 368.85 | 369.13 | 369.64 | 369.91 | 370.38 |
| pH | Caries | 6.40 | 6.65 | 7.05 | 7.60 | 7.80 |
| | No caries | 6.20 | 7.30 | 7.60 | 7.60 | 7.80 |
| Buffer capacity | Caries | 1.50 | 4.13 | 5.50 | 8.88 | 10.00 |
| | No caries | 4.00 | 5.00 | 6.25 | 8.75 | 11.00 |

pH: 0 to 6.5 = acid; 6.5 to 7.5 = neutral (saliva optimum); 7.5 to 14 basic.
Buffer capacity: 0 to 5 = very low; 0 to 6 = low; 10 to 12 = normal/high.

**Example 3:** *The multivariate SIMMA test*

Based on the above data, a Salivary Immune and Metabolic Marker Analysis test (SIMMA test) was proposed, wherein said SIMMA test is based on measuring the concentrations (salivary values) of 6 selected compounds in the saliva from any given individual (patient) at a given point in time, wherein of the 6 selected compounds, two are related to the immune competence, another two are related to the adhesion capacity of microorganisms, and another two are related to the acid production and buffering. Thus, the following presents two multivariate caries risk models, based on parameters of the SIMMA test, for saliva samples taken at two different times. In particular, in order to construct and evaluate the multivariate global risk model, the same study was carried out separately at 0.5 hours and 6 hours.

(1) By means of the 'genalg' library of the statistical package R and using accuracy (calculated by 50-fold cross-validation) as fitness, the best structure for the linear predictor of a logistic model (including the selection variables and their interactions), whereby the model having greatest interactions between variables was found.
(2) The accuracy (calculated by 50-fold cross validation) of this model was compared with the independence model (which only includes the selection variables in the linear predictor, and not the possible interactions between them).

* Variables selected at 0.5 hours:

("IgA_0.5", "LL.37_0.5", "Lactate_0.5", "Phosphate_0.5", "Statherin_0.5", "Fibronectin_0.5")

* Variables selected at 6 hours:

("Beta.2_6", "LL.37_6", "Formate_6", "Phosphate_6", "CollagenI_6", "Fibronectin_6")

In the representation of the models "y" is named the logit value of p {log[p / (1 - p)]}, and the model assumes that the response variable, which contains the status (CA or NOCA) of each individual, follows a binomial distribution with parameter p, and p corresponds to the probability for the response variable of taking value NOCA. The interpretation of the estimated model parameters (positive or negative interactions) is the following: when the value is positive, this means that an increase of the term associated with that parameter will increase the associated "y" value and, therefore, the p value, since the logit is an increasing function, and when the value is negative this means that an increase of the term associated with that parameter will decrease the associated "y" value and, therefore, the p value. Note that the risk of cavities has been defined as 1 - p, such that an increase in p means a reduced risk and a decrease in p implies an increased risk.

Best model with interactions found with 'genalg at 0.5 hours':

$$y = alph0 + alph1*LL.37\_0.5 + alph2*IgA\_0.5*Statherin\_0.5 +$$
$$alph3*LL.37\_0.5*Fibronectin\_0.5 + alph4*Lactate\_0.5*Phosphate\_0.5$$

alph0 = 4.194
alph1 = $-6.79 \times 10^{-2}$
alph2 = $5.19 \times 10^{-6}$
alph3 = $-5.37 \times 10^{-3}$
alph4 = $8.11 \times 10^{-3}$

To calculate the caries risk associated with a new observation of the variables:

$$\text{caries risk} = 1 - \{exp(y)/[1 + exp(y)]\}$$

Accuracy of the model with interactions found with 'genalg':

% Success in CA (true positive) = 100
% Success in NOCA (true negative) = 80

$$\text{Sensitivity} = \text{true positive} / (\text{true positive} + \text{false negative}) = 100/(100+20) = 83\%$$

$$\text{Specificity} = \text{true negative} / (\text{true negative} + \text{false positive}) = 80/(80+0) = 100\%$$

Distance between the real variable response and predicted risks in CA= 0.101
Distance between the real variable response and predicted risks in NOCA = 1
Based on the aforementioned model, the value of y for a saliva sample obtained from a subject CA 03 at 0.5 hours was calculated as follows:

$$y_{CA\,03} = 4.194 + (-6.79 \times 10^{-2} \times 126.87) + (5.19 \times 10^{-6} \times 56.09 \times 17973.00) +$$
$$(-5.37 \times 10^{-3} \times 126.87 \times 4.35) + (8.11 \times 10^{-3} \times 0.30 \times 46.61) = -2.04$$

Accordingly, the caries risk for subject CA 03 is:

$$\text{caries risk}_{CA\,03} = 1 - \{exp(-2.04)/[1 + exp(-2.04)]\} = 0.88$$

Thus, the subject CA 03 has a caries risk higher than the threshold value of 0.5, meaning that CA 03 is considered at risk of having or developing caries based on saliva sample composition at 0.5 hours (*i.e.* CA 03 affords a true positive result).

Analogously, the value for caries risk for a saliva sample obtained from a subject CA 01 at 0.5 hours was calculated as 0.19. Thus, the subject CA 01 has a caries risk lower than the threshold value of 0.5, meaning that subject CA 01 is not considered at risk of having or developing caries based on saliva sample composition at 0.5 hours (*i.e.* CA 01 affords a false negative result).

In contrast, the accuracy of the caries risk is comparatively much lower when calculated using an independence model (a model treating each of the six salivary component concentrations independently, not the possible interactions between them), as follows:

$$y = beta0 + beta1*IgA\_0.5 + beta2*LL.37\_0.5 + beta3*Lactate\_0.5 + beta4*Phosphate\_0.5 + beta5*Statherin\_0.5 + beta6*Fibronectin\_0.5$$

Accuracy of the independence model:

% Success in CA (true positive) = 70
% Success in NOCA (true negative) = 80

Distance between the real variable response and predicted risks in CA = 1.201

Distance between the real variable response and predicted risks in NOCA = 1

Best model with interactions found with 'genalg at 6 hours':

$$y = alph0 + alph1*CollagenI\_6 + alph2*Beta.2\_6*LL.37\_6 + alph3*LL.37\_6*Phosphate\_6 + alph4*Formate\_6*Fibronectin\_6$$

alph0 = 111.128
alph1 =-0.086
alph2 = -0.0029
alph3 = -0.0052
alph4 = -4.07

As before, to calculate the caries risk associated with a new observation of the variables:

$$caries\ risk = 1 - \{exp(y)/[1 + exp(y)]\}$$

Accuracy of the model with interactions found with 'genalg':
% Success in CA (true positive) = 100
% Success in NOCA (true negative) = 100

Distance between the real variable response and predicted risks in CA = $6.001 \times 10^{-6}$.

Distance between the real variable response and predicted risks in NOCA = $7.247 \times 10^{-5}$.

Based on the aforementioned model, the value of y for a saliva sample obtained from a subject CA 03 at 6 h was calculated as follows:

$$y_{CA\,03} = 111.128 + (-0.086 \times 23.80) + (-0.0029 \times 399.94 \times 162.91) +$$
$$(-0.0052 \times 162.91 \times 0.45) + (-4.07 \times 0.02 \times 3.12) = -80.51$$

Accordingly, the caries risk for subject CA 03 is:

$$\text{caries risk}_{CA\,03} = 1 - \{\exp(-80.51)/[1 + \exp(-80.51)]\} = 1$$

Thus, the subject CA 03 has a caries risk higher than the threshold value of 0.5, meaning that CA 03 is considered at risk of having or developing caries based on saliva sample composition at 6 hours.

In contrast, the accuracy of the caries risk is much lower when calculated using an independence model, as follows:

$$y = beta0 + beta1*Beta.2\_6 + beta2*LL.37\_6 + beta3*Formate\_6 +$$
$$beta4*Phosphate\_6 + beta5*CollagenI\_6 + beta6*Fibronectin\_6$$

Accuracy of the independence model:
% Success in CA (true positive) = 70
% Success in NOCA (true negative) = 80

$$\text{Distance between the real variable response and predicted risks in CA} = 1.001$$

$$\text{Distance between the real variable response and predicted risks in NOCA} = 0.803$$

Best model with interactions found with 'galgo at 6 hours':

In addition, the galgo method was used to obtain values for specificity and sensitivity.

The difference between the galgo method and the genalg method is that galgo makes the calculations taking into account all the evaluated models where the six selected variables are involved, whereas in the aforementioned genalg method only a single model is evaluated. Consequently, the galgo method is more robust and alleviates over-fitting.

The "Cross Validation" technique using the galgo method for data sampled at 6 hours afforded a sensitivity of 98% and a specificity of 88%. In other words, almost 100% of subjects with caries are classified correctly, whereas only 12% of subjects without caries are falsely assigned to the high caries risk group. It is thought that 12% of false positives arise within the group of subjects without caries because these subjects in fact have high caries risk, but have not clinically developed this condition due to, for example, the quality of their diet and/or oral hygiene. As a consequence, the method of the invention indicates that such subjects should take care of, for example, their oral hygiene since, in the absence of this they may develop caries.

Classification power:

In order to test whether any combination of concentrations of any of the aforementioned 24 compounds present in saliva may improve caries risk prediction, the statistical classification power between the 6 variables selected for the invention and 1000 random selections of variables was compared. Power may be defined as [proportion of correct classification of caries individuals (CA)] + [proportion of correct classification of caries-free individuals (NOCA)]. Thus, the maximum classification power value is 2. In order to estimate these proportions, a 50-fold cross validation approach was used. However, when the potential biomarkers of caries risk were randomly selected in groups of 6, the combinations did not improve the diagnostic value provided by the invention, neither at 0.5 hours after brushing teeth **(Figure 10A)** nor at 6 hours **(Figure 10B).** Specifically, the median classification power of the 6 randomly selected compounds was 1.2 at both 0.5 and 6 hours. Thus, it may be concluded that it is not obvious for an expert in the field, based on the data available in the literature, to select the 6 compounds included in the current invention, that are the ones that maximize the diagnostic value of the measured biomarkers.

**Example 4:** *The univariate SIMMA test*

Based on the fact that the results from the multivariate analysis indicate that subject CA 03 exhibits a high caries risk, a univariate SIMMA test was proposed, wherein each of said salivary concentrations employed in the multivariate tests of Example 3 are then compared to the corresponding reference values obtained from a healthy population of a similar age **(Figures 7** and **8** show the reference values for adults). The concentrations falling outside the healthy range are indicative of caries risk due to an imbalance in the corresponding category.

The following exemplifies the univariate application of the SIMMA method of the invention in the diagnosis of caries risk in a subject using the concentration of cathelicidin LL-37 in a saliva sample taken 6 hours after brushing teeth. Using ELISA analysis, the concentration (ng/mL) of cathelicidin LL-37 in the sample was previously determined as 162.91 ng/mL (*cf.* **Table 5B).**

A) The concentration of cathelicidin LL-37 of CA 03 of 162.91 ng/mL (*cf.* **Table 5B)** is greater than the threshold concentration value of 103 ng/mL which was determined from the sampled population of healthy (non-caries) subjects (n = 10, median = 91.6 ng/mL, interquartile range = 88.5 - 103 ng/mL, cf. **Figure 4G),** wherein the threshold value is based on a specificity of more than 75%. In fact, the concentration of cathelicidin LL-37 of CA 01 of 162.91 ng/mL was found to fall outside 3 standard deviations of the mean of the concentration of cathelicidin LL-37 of healthy (non-caries) subjects *(i.e.* there is less than 1% probability that CA 03 belongs to the population of healthy patients).

B) The concentration of cathelicidin LL-37 of CA 03 of 162.91 ng/mL (*cf.* **Table 5B)** is far greater than the threshold concentration value of 130 ng/mL which was determined from the sampled population of subjects with caries (n = 10, mean = 139 ng/mL, interquartile range = 130 - 163 ng/mL, *cf.* **Figure 4B),** wherein the threshold value is based on a sensitivity of more than 75%.

Consequently, using either form of comparative analysis, A) or B), the subject CA 03 was determined to be at risk of developing or having caries due to an imbalance in at least the salivary immune system protein cathelicidin LL-37 in a sample obtained 6 hours after brushing teeth, and was referred to a specialist for appropriate treatment of at least said imbalance.

Thus, the SIMMA test provides not only a general caries risk assessment, but also identifies the likely biological origin of said risk, namely whether it derives from an immune imbalance, and/or tendency to adhesion of cariogenic organisms, and/or lack of acid buffering.

**REFERENCES**

Aas J.A., Paster B.J., Stokes L.N., Olsen I., Dewhirst F.E.: Defining the normal bacterial flora of the oral cavity. J. Clin. Microbiol. 2005; 43:5721 - 5732.

Aas J.A., Griffen A.L., Dardis S.R., Lee A.M., Olsen I., Dewhirst F.E., et al.: Bacteria of dental caries in primary and permanent teeth in children and young adults. J. Clin. Microbiol. 2008; 46: 1407 - 1417.

Belda-Ferre P., Alcaraz L.D., Cabrera-Rubio R., Romero H., Simón-Soro A., Pignatelli M., et al.: The oral metagenome in health and disease; ISME J 2012; 6:46 - 56.

Vincent Calcagno (2013). glmulti: Model selection and multimodel inference made easy. R package version 1.0.7. http://CRAN.R-project.org/package=glmulti

Crielaard W., Zaura E., Schuller A.A., Huse S.M., Montijn R.C., et al.: Exploring the oral microbiota of children at various developmental stages of their dentition in the relation to their oral health; BMC Med. Genomics 2011; 4: 22.

Guo L., Shi W.: Salivary biomarkers for caries risk assessment; J. Calif. Dent. Assoc. 2013; 41(2):107-109, 112 - 118.

Keyes P.H., Jordan H.V.: Factors influencing initiation, transmission and inhibition of dental caries. In: Harris R.J.,

ed. Mechanisms of hard tissue destruction. New York: Academic Press 1963; :261 - 283.

Loë H., Silness J.: Periodontal disease in pregnancy. Acta Odontologica. Scandinavica 1963; 21, 533.

Navazesh M., Christensen C.M.: A comparison of whole mouth resting and stimulated salivary measuremen procedures. J. Dent. Res. 1982; 61:1158 - 1162

Olson et al: Use of 16S ribosomal RNA gene analyses to characterize the bacterial signature associated with poor oral health in West Virginia; BMC Oral Health 2011; 11:7.

Petersen P.E.: Challenges to improvement of oral health in the 21st century--the approach of the WHO Global Oral Health Programme; Int. Dent. J. 2004; 54(6 Suppl 1):329 - 343.

Plonka K.A., Pukallus M.L., Barnett A.G., Walsh L.J., Holcombe T.H., Seow W.K.: Mutans streptococci and lactobacilli colonization in predentate children from the neonatal period to seven months of age; Caries Res. 2012; 46(3):213 - 220.

R Core Team (2014). R: A language and environment for statistical computing; R Foundation for Statistical Computing, Vienna, Austria. URL http://www.R-project.org/.

Schafer C.A., Schafer J.J., Yakob M., Lima P., Camargo P., Wong D.T.: Saliva diagnostics: utilizing oral fluids to determine health status; Monogr. Oral Sci. 2014; 24:88 - 98.

Shah A.G., Shetty P.C., Ramachandra C.S., Bhat N.S., Laxmikanth S.M.: In vitro assessment of photocatalytic titanium oxide surface modified stainless steel orthodontic brackets for antiadherent and antibacterial properties against Lactobacillus acidophilus; Angle Orthod. 2011; 81:1028 - 1035.

Simon-Soro A., Tomás I., Cabrera-Rubio R., Catalan M.D., Nyvad B., Mira A.: Microbial geography of the oral cavity; J. Dent. Res. 2013 Jul; 92(7): 616 - 621.

Simón-Soro A., Belda-Ferre P., Cabrera-Rubio R., Alcaraz L.D., Mira A.: A tissue-dependent hypothesis of dental caries. Caries Res. 2013; 47(6):591 - 600.

Takahashi N., Nyvad B.: The Role of Bacteria in the Caries Process: Ecological Perspectives. J. Dent. Res. 2011; 90:294 - 303.

Trevino V., Falciani F. (2014). galgo: Genetic Algorithms for Multivariate Statistical Models from Large-scale Functional Genomics Data. R package version 1.2; http://bioinformatica.mty.itesm.mx/?q=node/82

Venables, W.N., Ripley, B.D. (2002) Modern Applied Statistics with S; 4th Ed. Springer, New York. ISBN 0-387-95457-0

World Health Organization. Oral Health Surveys- Basic Methods; 4th Ed. Geneva: World Health Organization 1997.

Yoshizawa J.M., Schafer C.A., Schafer J.J., Farrell J.J., Paster B.J., Wong D.T.: Salivary biomarkers: Toward future clinical and diagnostic utilities; Clin. Microbiol. Rev. 2013; 26:781 - 791.

**Claims**

1. A method for predicting caries risk in a subject, **characterized in that** said method comprises the following steps:

    (a) determining the concentration in saliva from said subject of

        i) at least one compound selected from salivary components of the immune system; and
        ii) at least one compound selected from salivary components involved in microbial adhesion to a surface of the buccal cavity; and
        iii) at least one compound selected from salivary components involved in metabolism in the buccal cavity or in regulating the pH of the buccal cavity;

(b) comparing a response variable with a corresponding reference response variable in a population of subjects without caries or comparing a response variable with a corresponding reference response variable in a population of subjects with caries, wherein a response variable is calculated from more than one concentration determined in step (a); and
(c) determining the caries risk based on the degree of difference in response variable compared in step (b), wherein

iv) a greater degree of difference between the response variable of the subject and the corresponding reference response variable in a population of subjects without caries, or
v) a smaller degree of difference between the response variable of the subject and the corresponding reference response variable in a population of subjects with caries,
is indicative of higher caries risk in said subject.

2. The method according to claim 1, which further comprises prescribing a plan of care to a subject determined to have a high caries risk in step (c), wherein said plan of care comprises

(d) comparing at least one concentration used to calculate the response variable compared in step (b) with a corresponding reference concentration in a population of subjects without caries, or comparing at least one concentration used to calculate the response variable compared in step (b) with a corresponding reference concentration in a population of subjects with caries; and
(e) determining the imbalance in said concentration based on the degree of difference in concentration compared in step (d), wherein

(vi) a greater degree of difference between the concentration of the subject and the corresponding reference concentration in a population of subjects without caries, or
(vii) a smaller degree of difference between the concentration of the subject and the corresponding reference concentration in a population of subjects with caries,
is indicative of higher imbalance in said concentration; and

(f) modulating

(viii) the immune system, when a compound whose concentration is determined to have a high imbalance in step (e) is a compound selected from salivary components of the immune system;
(ix) microbial adhesion to a surface of the buccal cavity, when a compound whose concentration is determined to have a high imbalance in step (e) is a compound selected from salivary components involved in microbial adhesion to a surface of the buccal cavity; and/or
(x) metabolism in the buccal cavity or the pH of the buccal cavity, when a compound whose concentration is determined to have a high imbalance in step (e) is a compound selected from salivary components involved in metabolism in the buccal cavity or in regulating the pH of the buccal cavity.

3. The method according to claims 1 or 2, wherein

- the at least one compound selected from salivary components of the immune system is at least one compound selected from immunoglobulins, an antimicrobial peptide and/or a protein of the complement system;
- the at least one compound selected from salivary components involved in microbial adhesion to a surface of the buccal cavity is at least one compound selected from salivary components to which microorganisms adhere; and
- the at least one compound selected from salivary components involved in metabolism in the buccal cavity or in regulating the pH of the buccal cavity is at least one compound selected from a buffering agent, an agent that neutralizes acids, an enzyme that metabolizes at least one sugar and/or a compound produced during fermentation.

4. The method according to claim 3, wherein

- the at least one compound selected from salivary components of the immune system is at least one compound selected from the list comprising Cathelicidin LL-37, IgA, Alpha-Defensin 1-3, IgM, Beta-Defensin 1, Lactoferrin, C3a, Calprotectin, Beta-Defensin 3, IgG, Beta-Defensin 2 and Lysozyme;
- the at least one compound selected from salivary components involved in microbial adhesion to a surface of the buccal cavity is at least one compound selected from the list comprising Statherin, Fibronectin, Mucin 5B, Mucin 7, Collagen I, Alpha-2 Macroglobulin and PRB1; and
- the at least one compound selected from salivary components involved in metabolism in the buccal cavity or in regulating the pH of the buccal cavity is at least one compound selected from the list comprising Phosphate, Lactate, Urea, Calcium and Formate.

5. The method according to claim 4, wherein

- the at least one compound selected from salivary components of the immune system is at least one compound selected from the list comprising Cathelicidin LL-37, IgA and Beta-Defensin 2;
- the at least one compound selected from salivary components involved in microbial adhesion to a surface of the buccal cavity is at least one compound selected from the list comprising Statherin, Fibronectin and Collagen I; and
- the at least one compound selected from salivary components involved in metabolism in the buccal cavity or in regulating the pH of the buccal cavity is at least one compound selected from the list comprising Phosphate, Lactate and Formate.

6. The method according to claim 5, wherein

- the at least one compound selected from salivary components of the immune system is at least one compound selected from the list comprising Cathelicidin LL-37 and IgA;
- the at least one compound selected from salivary components involved in microbial adhesion to a surface of the buccal cavity is at least one compound selected from the list comprising Statherin and Fibronectin; and
- the at least one compound selected from salivary components involved in metabolism in the buccal cavity or in regulating the pH of the buccal cavity is at least one compound selected from the list comprising Phosphate and Lactate, and
the saliva used for determining the concentration of at least one compound selected from salivary components of the immune system, and of at least one compound selected from salivary components involved in microbial adhesion to a surface of the buccal cavity is obtained at a time (a1) of duration z after brushing teeth, and/or at a time (b1) of a duration y after brushing teeth; and the saliva used for determining the concentration of at least one compound selected from salivary components involved in metabolism in the buccal cavity or in regulating the pH of the buccal cavity is obtained:

i) at a time (a2) coincident with the aforementioned time (a1) and a time of a duration x after swilling with a sucrose solution, and/or at a time (b2) coincident with the aforementioned time (b1) and a time of said duration x after swilling with a second sucrose solution, wherein swilling with a sucrose solution is performed after brushing teeth; or

ii) at a time (a3) coincident with a time of said duration z prior to the aforementioned time (a1) (*i.e.* immediately prior to brushing teeth) and a time of said duration x after swilling with a sucrose solution, and/or at a time (b3) coincident with a time of said duration z prior to the aforementioned time (b1) and a time of said duration x after swilling with a sucrose solution; or

iii) at a time (c2) of said duration x after swilling with a sucrose solution, wherein swilling with a sucrose solution is performed immediately after obtaining the saliva sample obtained at time (a1) and/or time (b1), wherein duration z is between 0 and 2 hours, duration y is between 2 and 24 hours and duration x is between 0 and 20 minutes.

7. The method according to claim 5, wherein

- the at least one compound selected from salivary components of the immune system is at least one compound selected from the list comprising Cathelicidin LL-37 and Beta-Defensin 2;
- the at least one compound selected from salivary components involved in microbial adhesion to a surface of the buccal cavity is at least one compound selected from the list comprising Fibronectin and Collagen I; and
- the at least one compound selected from salivary components involved in metabolism in the buccal cavity or in regulating the pH of the buccal cavity is at least one compound selected from the list comprising Phosphate and Formate, and
the saliva is obtained at a time (a) coincident with between 0 and 2 hours after brushing teeth and between 0 and 20 minutes after swilling with a sucrose solution, and/or at a time (b) coincident with between 2 and 24 hours after brushing teeth and between 0 and 20 minutes after swilling with a second sucrose solution, wherein each step of swilling with a sucrose solution is performed after brushing teeth.

8. A kit for predicting caries risk in a subject **characterized in that** said kit comprises:

   (a) means for determining the concentration in saliva from said subject of

   > i) at least one compound selected from salivary components of the immune system; and
   > ii) at least one compound selected from salivary components involved in microbial adhesion to a surface of the buccal cavity; and
   > iii) at least one compound selected from salivary components involved in metabolism in the buccal cavity or in regulating the pH of the buccal cavity;

   (b) instructions for comparing a response variable with a corresponding reference response variable in a population of subjects without caries or comparing a response variable with a corresponding reference response variable in a population of subjects with caries, wherein a response variable is calculated from more than one concentration determined in step (a); and
   (c) instructions for determining the caries risk based on the degree of difference in response variable compared in step (b), wherein

   > iv) a greater degree of difference between the response variable of the subject and the corresponding reference response variable in a population of subjects without caries, or
   > v) a smaller degree of difference between the response variable of the subject and the corresponding reference response variable in a population of subjects with caries,
   > is indicative of higher caries risk in said subject.

9. The kit according to claim 8, which further comprises instructions for prescribing a plan of care to a subject determined to have a high caries risk in step (c), wherein said plan of care comprises

   > (d) comparing at least one concentration used to calculate the response variable compared in step (b) with a corresponding reference concentration in a population of subjects without caries, or comparing at least one concentration used to calculate the response variable compared in step (b) with a corresponding reference concentration in a population of subjects with caries; and
   > (e) determining the imbalance in said concentration based on the degree of difference in concentration compared in step (d), wherein

   >> (vi) a greater degree of difference between the concentration of the subject and the corresponding reference concentration in a population of subjects without caries, or
   >> (vii) a smaller degree of difference between the concentration of the subject and the corresponding reference concentration in a population of subjects with caries,
   >> is indicative of higher imbalance in said concentration; and

   > (f) modulating

   >> (viii) the immune system, when a compound whose concentration is determined to have a high imbalance in step (e) is a compound selected from salivary components of the immune system;
   >> (ix) microbial adhesion to a surface of the buccal cavity, when a compound whose concentration is determined to have a high imbalance in step (e) is a compound selected from salivary components involved in microbial adhesion to a surface of the buccal cavity; and/or
   >> (x) metabolism in the buccal cavity or the pH of the buccal cavity, when a compound whose concentration is determined to have a high imbalance in step (e) is a compound selected from salivary components involved in metabolism in the buccal cavity or in regulating the pH of the buccal cavity.

10. The kit according to claims 8 or 9, wherein

   - the at least one compound selected from salivary components of the immune system is at least one compound selected from immunoglobulins, an antimicrobial peptide and/or a protein of the complement system;

- the at least one compound selected from salivary components involved in microbial adhesion to a surface of the buccal cavity is at least one compound selected from salivary components to which microorganisms adhere; and
- the at least one compound selected from salivary components involved in metabolism in the buccal cavity or in regulating the pH of the buccal cavity is at least one compound selected from a buffering agent, an agent that neutralizes acids, an enzyme that metabolizes at least one sugar and/or a compound produced during fermentation.

**11.** The kit according to claim 10, wherein

- the at least one compound selected from salivary components of the immune system is at least one compound selected from the list comprising Cathelicidin LL-37, IgA, Alpha-Defensin 1-3, IgM, Beta-Defensin 1, Lactoferrin, C3a, Calprotectin, Beta-Defensin 3, IgG, Beta-Defensin 2 and Lysozyme;
- the at least one compound selected from salivary components involved in microbial adhesion to a surface of the buccal cavity is at least one compound selected from the list comprising Statherin, Fibronectin, Mucin 5B, Mucin 7, Collagen I, Alpha-2 Macroglobulin and PRB1; and
- the at least one compound selected from salivary components involved in metabolism in the buccal cavity or in regulating the pH of the buccal cavity is at least one compound selected from the list comprising Phosphate, Lactate, Urea, Calcium and Formate.

**12.** The kit according to claim 11, wherein

- the at least one compound selected from salivary components of the immune system is at least one compound selected from the list comprising Cathelicidin LL-37, IgA and Beta-Defensin 2;
- the at least one compound selected from salivary components involved in microbial adhesion to a surface of the buccal cavity is at least one compound selected from the list comprising Statherin, Fibronectin and Collagen I; and
- the at least one compound selected from salivary components involved in metabolism in the buccal cavity or in regulating the pH of the buccal cavity is at least one compound selected from the list comprising Phosphate, Lactate and Formate.

**13.** The kit according to claim 12, wherein

- the at least one compound selected from salivary components of the immune system is at least one compound selected from the list comprising Cathelicidin LL-37 and IgA;
- the at least one compound selected from salivary components involved in microbial adhesion to a surface of the buccal cavity is at least one compound selected from the list comprising Statherin and Fibronectin; and
- the at least one compound selected from salivary components involved in metabolism in the buccal cavity or in regulating the pH of the buccal cavity is at least one compound selected from the list comprising Phosphate and Lactate, and

the saliva used for determining the concentration of at least one compound selected from salivary components of the immune system, and of at least one compound selected from salivary components involved in microbial adhesion to a surface of the buccal cavity is obtained at a time (a1) of duration z after brushing teeth, and/or at a time (b1) of a duration y after brushing teeth; and the saliva used for determining the concentration of at least one compound selected from salivary components involved in metabolism in the buccal cavity or in regulating the pH of the buccal cavity is obtained:

i) at a time (a2) coincident with the aforementioned time (a1) and a time of a duration x after swilling with a sucrose solution, and/or at a time (b2) coincident with the aforementioned time (b1) and a time of said duration x after swilling with a second sucrose solution, wherein swilling with a sucrose solution is performed after brushing teeth; or
ii) at a time (a3) coincident with a time of said duration z prior to the aforementioned time (a1) (*i.e.* immediately prior to brushing teeth) and a time of said duration x after swilling with a sucrose solution, and/or at a time (b3) coincident with a time of said duration z prior to the aforementioned time (b1) and a time of said duration x after swilling with a sucrose solution; or
iii) at a time (c2) of said duration x after swilling with a sucrose solution, wherein swilling with a sucrose solution is performed immediately after obtaining the saliva sample obtained at time (a1) and/or time (b1), wherein duration z is between 0 and 2 hours, duration y is between 2 and 24 hours and duration x is between 0 and 20 minutes.

14. The kit according to claim 12, wherein

- the at least one compound selected from salivary components of the immune system is at least one compound selected from the list comprising Cathelicidin LL-37 and Beta-Defensin 2;
- the at least one compound selected from salivary components involved in microbial adhesion to a surface of the buccal cavity is at least one compound selected from the list comprising Fibronectin and Collagen I; and
- the at least one compound selected from salivary components involved in metabolism in the buccal cavity or in regulating the pH of the buccal cavity is at least one compound selected from the list comprising Phosphate and Formate, and
the saliva is obtained at a time (a) coincident with between 0 and 2 hours after brushing teeth and between 0 and 20 minutes after swilling with a sucrose solution, and/or at a time (b) coincident with between 2 and 24 hours after brushing teeth and between 0 and 20 minutes after swilling with a second sucrose solution, wherein each step of swilling with a sucrose solution is performed after brushing teeth.

15. Use of the method according to any of claims 1 to 7 or the kit according to any of claims 8 to 14 in predicting caries risk in a subject.

**Figure 1**

**Figure 1 (cont.)**

**G**  Cathelicidin LL.37_0.5

**H**  C3a_0.5

Calprotectin_0.5

**J**  Lactoferrin_0.5

**K**  Alpha-Defensin 1-3_0.5

**L**  Lysozyme_0.5

**Figure 2**

A     Fibronectin_0.5

B     Collagen I_0.5

C     Statherin_0.5

D     Mucin 7_0.5

E     Mucin 5B_0.5

F     PRB1_0.5

**Figure 2 (cont.)**

**G**      Alpha-2 Macroglobulin_0.5

**Figure 3**

A — Formate_0.5
B — Phosphate_0.5
C — Lactate_0.5
D — Calcium_0.5
E — Urea_0.5

**Figure 4**

**Figure 4 (cont.)**

G    Cathelicidin LL.37_6

H    C3a_6

Calprotectin_6

J    Lactoferrin_6

K    Alpha-Defensin 1-3_6

L    Lysozyme_6

**Figure 5**

A    Fibronectin_6

B    Collagen I_6

C    Statherin_6

D    Mucin 7_6

E    Mucin 5B_6

F    PRB1_6

**Figure 5 (cont.)**

**G**     Alpha 2 Macroglobulin_6

**Figure 6**

**Figure 7**

## Salivary adhesion components

A — Fibronectin_0
B — Statherin_0

## Salivary acidity-regulating components

C — Formate_0
D — Phosphate_0

## Salivary immune system components

E — IgA_0
F — Cathelicidin LL.37_0

44

**Figure 8**

Salivary adhesion components

A  Fibronectin_6
B  Collagen I_6

Salivary acidity-regulating components

C  Formate_6
D  Phosphate_6

Salivary immune system components

E  Cathelicidin LL.37_6
F  Beta.2_6

**Figure 9**

**A** IgA

**B** pH

**Figure 9 (cont.)**

C                                                    Buffer capacity

**Figure 10**

A

B

**Figure 11**

Europäisches
Patentamt

European
Patent Office

Office européen
des brevets

# EUROPEAN SEARCH REPORT

Application Number

EP 14 19 5383

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NORDLUND NOT AKE ET AL: "Improved ability of biological and previous caries multimarkers to predict caries disease as revealed by multivariate PLS modelling", BMC ORAL HEALTH, BIOMED CENTRAL, LONDON, GB, vol. 9, no. 1, 3 November 2009 (2009-11-03), page 28, XP021063010, ISSN: 1472-6831, DOI: 10.1186/1472-6831-9-28 | 1-7 | INV. G01N33/68 G01N33/84 |
| A | DENNY ET AL: "A novel saliva test for caries risk assessment", JOURNAL OF THE CALIFORNIA DENTAL ASSOCIATION, THE ASSOCIATION, SACRAMENTO, CA, US, vol. 34, no. 4, 1 April 2005 (2005-04-01), page 287, XP009074177, ISSN: 1043-2256 | 1-7 | |
| A | DALE BEVERLY A ET AL: "Oral Antimicrobial Peptides and Biological Control of Caries", BMC ORAL HEALTH, BIOMED CENTRAL, LONDON, GB, vol. 6, no. Suppl 1, 15 June 2006 (2006-06-15), page S13, XP021017246, ISSN: 1472-6831, DOI: 10.1186/1472-6831-6-S1-S13 | 1-7 | TECHNICAL FIELDS SEARCHED (IPC) G01N |

-/--

The present search report has been drawn up for all claims

| Place of search | Date of completion of the search | Examiner |
|---|---|---|
| Munich | 7 April 2015 | Behrens, Ralf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

| | Europäisches Patentamt<br>European Patent Office<br>Office européen des brevets | **EUROPEAN SEARCH REPORT** | Application Number<br>EP 14 19 5383 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KIRSTILA V ET AL: "LONGITUDINAL ANALYSIS OF THE ASSOCIATION OF HUMAN SALIVARY ANTIMICROBIAL AGENTS WITH CARIES INCREMENT AND CARIOGENIC MICRO-ORGANISMS: A TWO-YEAR COHORT STUDY",<br>JOURNAL OF DENTAL RESEARCH, INTERNATIONAL ASSOCIATION FOR DENTAL RESEARCH, US,<br>vol. 77, no. 1,<br>1 January 1998 (1998-01-01), pages 73-80,<br>XP008008833,<br>ISSN: 0022-0345 | 1-7 | |

TECHNICAL FIELDS
SEARCHED (IPC)

~~The present search report has been drawn up for all claims~~

| Place of search | Date of completion of the search | Examiner |
|---|---|---|
| Munich | 7 April 2015 | Behrens, Ralf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt
European
Patent Office
Office européen
des brevets

Application Number

EP 14 19 5383

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-7, 15(all partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

Application Number

EP 14 19 5383

The Search Division considers that the present European patent application does not comply with the
requirements of unity of invention and relates to several inventions or groups of inventions, namely:

1. claims: 1-7, 15(all partially)

Method of claim 4, wherein the compound of the immune system
is IgA, the microbial adhesion compound is statherin, and
the metabolic / pH compound is phosphate.
---

2. claims: 1-7, 15(all partially)

Method of claim 4, wherein the compound of the immune system
is IgB, the microbial adhesion compound is statherin, and
the metabolic / pH components is phosphate.
---

3-999. claims: 1-7, 15(all partially)

Method of claim 4, wherein the compounds of the immune
system, of microbial adhesion and of metabolism / regulating
pH are other combinations of compounds selected from the
list of compounds of claim 4.
---

1000. claims: 8-14(completely); 15(partially)

Kit according to claim 8.
---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AAS J.A. ; PASTER B.J. ; STOKES L.N. ; OLSEN I. ; DEWHIRST F.E.** Defining the normal bacterial flora of the oral cavity. *J. Clin. Microbiol.,* 2005, vol. 43, 5721-5732

- **AAS J.A. ; GRIFFEN A.L. ; DARDIS S.R. ; LEE A.M. ; OLSEN I. ; DEWHIRST F.E. et al.** Bacteria of dental caries in primary and permanent teeth in children and young adults. *J. Clin. Microbiol.,* 2008, vol. 46, 1407-1417

- **BELDA-FERRE P. ; ALCARAZ L.D. ; CABRERA-RUBIO R. ; ROMERO H. ; SIMÓN-SORO A. ; PIGNATELLI M. et al.** The oral metagenome in health and disease. *ISME J,* 2012, vol. 6, 46-56

- **VINCENT CALCAGNO.** glmulti: Model selection and multimodel inference made easy. *R package version 1.0.7.,* 2013, http://CRAN.R-project.org/package=glmulti

- **CRIELAARD W. ; ZAURA E. ; SCHULLER A.A. ; HUSE S.M. ; MONTIJN R.C. et al.** Exploring the oral microbiota of children at various developmental stages of their dentition in the relation to their oral health. *BMC Med. Genomics,* 2011, vol. 4, 22

- **GUO L. ; SHI W.** Salivary biomarkers for caries risk assessment. *J. Calif. Dent. Assoc.,* 2013, vol. 41 (2), 107-109, 112-118

- Factors influencing initiation, transmission and inhibition of dental caries. **KEYES P.H. ; JORDAN H.V.** Mechanisms of hard tissue destruction. Academic Press, 1963, 261-283

- **LOË H. ; SILNESS J.** Periodontal disease in pregnancy. *Acta Odontologica. Scandinavica,* 1963, vol. 21, 533

- **NAVAZESH M. ; CHRISTENSEN C.M.** A comparison of whole mouth resting and stimulated salivary measuremen procedures. *J. Dent. Res.,* 1982, vol. 61, 1158-1162

- **OLSON et al.** Use of 16S ribosomal RNA gene analyses to characterize the bacterial signature associated with poor oral health in West Virginia. *BMC Oral Health,* 2011

- **PETERSEN P.E.** Challenges to improvement of oral health in the 21st century--the approach of the WHO Global Oral Health Programme. *Int. Dent. J.,* 2004, vol. 54 (6), 329-343

- **PLONKA K.A. ; PUKALLUS M.L. ; BARNETT A.G. ; WALSH L.J. ; HOLCOMBE T.H. ; SEOW W.K.** Mutans streptococci and lactobacilli colonization in predentate children from the neonatal period to seven months of age. *Caries Res.,* 2012, vol. 46 (3), 213-220

- **R CORE TEAM.** R: A language and environment for statistical computing. *R Foundation for Statistical Computing,* 2014, http://www.R-project.org

- **SCHAFER C.A. ; SCHAFER J.J. ; YAKOB M. ; LIMA P. ; CAMARGO P. ; WONG D.T.** Saliva diagnostics: utilizing oral fluids to determine health status. *Monogr. Oral Sci.,* 2014, vol. 24, 88-98

- **SHAH A.G. ; SHETTY P.C. ; RAMACHANDRA C.S. ; BHAT N.S. ; LAXMIKANTH S.M.** In vitro assessment of photocatalytic titanium oxide surface modified stainless steel orthodontic brackets for antiadherent and antibacterial properties against Lactobacillus acidophilus. *Angle Orthod.,* 2011, vol. 81, 1028-1035

- **SIMON-SORO A. ; TOMÁS I. ; CABRERA-RUBIO R. ; CATALAN M.D. ; NYVAD B. ; MIRA A.** Microbial geography of the oral cavity. *J. Dent. Res.,* July 2013, vol. 92 (7), 616-621

- **SIMÓN-SORO A. ; BELDA-FERRE P. ; CABRERA-RUBIO R. ; ALCARAZ L.D. ; MIRA A.** A tissue-dependent hypothesis of dental caries. *Caries Res.,* 2013, vol. 47 (6), 591-600

- **TAKAHASHI N. ; NYVAD B.** The Role of Bacteria in the Caries Process: Ecological Perspectives. *J. Dent. Res.,* 2011, vol. 90, 294-303

- **TREVINO V. ; FALCIANI F.** galgo: Genetic Algorithms for Multivariate Statistical Models from Large-scale Functional Genomics Data. *R package version 1.2,* 2014, http://bioinformatica.mty.itesm.mx/?q=node/82

- **VENABLES, W.N. ; RIPLEY, B.D.** Modern Applied Statistics with S. Springer, 2002

- Oral Health Surveys- Basic Methods. World Health Organization, 1997

- **YOSHIZAWA J.M. ; SCHAFER C.A. ; SCHAFER J.J. ; FARRELL J.J. ; PASTER B.J. ; WONG D.T.** Salivary biomarkers: Toward future clinical and diagnostic utilities. *Clin. Microbiol. Rev.,* 2013, vol. 26, 781-791